(19) Europäisches Patentamt / European Patent Office / Office européen des brevets

(11) **EP 3 021 802 B1**

(12) **EUROPEAN PATENT SPECIFICATION**

(45) Date of publication and mention
of the grant of the patent:
**12.06.2019 Bulletin 2019/24**

(21) Application number: **15753739.0**

(22) Date of filing: **21.07.2015**

(51) Int Cl.:
*A61F 7/00* *(2006.01)*    *A61H 33/00* *(2006.01)*

(86) International application number:
**PCT/GB2015/052103**

(87) International publication number:
**WO 2016/016611 (04.02.2016 Gazette 2016/05)**

(54) **THERMOREGULATION SYSTEM AND METHOD**

THERMOREGLUNGSSYSTEM UND VERFAHREN

SYSTÈME ET MÉTHODE DE THERMORÉGULATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.07.2014 GB 201413601
26.03.2015 GB 201505218**

(43) Date of publication of application:
**25.05.2016 Bulletin 2016/21**

(73) Proprietor: **Physiolab Technologies Limited
Colchester
Essex C03 3BZ (GB)**

(72) Inventor: **ROSE, Nicholas
Colchester
Essex CO3 3BZ (GB)**

(74) Representative: **Williams Powell
11 Staple Inn
London WC1V 7QH (GB)**

(56) References cited:
**US-A1- 2005 065 581    US-A1- 2005 103 353
US-B1- 6 319 205**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to thermoregulation systems and assemblies for personal use, for example for applying a thermal treatment to a patient in order to alleviate or cure physical injuries and disorders, and methods therefor.

Background

There are numerous instances where it is desired to effect a thermal treatment on a patient. For example, this may be to treat a physical injury, such as of the muscles, ligaments, tendons and the like. It may also be useful in treating skin injuries, as well as illnesses such as infections and so on as well as to accelerate natural recovery.

Thermal treatments of this type have been known for many years, in their simplest form being ice packs.

More recently, thermoregulation devices have been developed which provide built-in control, for example in which fluid is provided to a cuff or pad with particular feed parameters in order to provide a desired energy transfer to a patient. Examples are disclosed in WO 2013/190337 and WO 2013/190336.

US2005/0103353 discloses controlled heat transfer with mammalian bodies.

Summary of the Present Invention

The present invention seeks to provide an improved thermoregulation system, assembly, and method.

The invention is defined in appended independent claims 1 and 7, preferred embodiments are described in the dependent claims.

According to an aspect of the invention, there is provided a method of determining an operational parameter for thermoregulation fluid to be provided for a personal thermoregulation treatment pack, the pack including a thermoregulation zone for at least partially surrounding a treatment zone and for applying a thermal treatment to a user at the treatment zone, the method including:

measuring a first volume indicator, the first volume indicator being indicative of a volume of the treatment zone when occupied by a first user;

determining, from the first volume indicator, a first operational parameter for thermoregulation fluid to be provided for treating the first user, characterized in that the first operational parameter is a volumetric parameter.

In preferred embodiments, the first volume indicator is a measure of the volume of the treatment zone when occupied by the first user. However, as described below, the first volume indicator can in some embodiments be other indicators of the volume of the treatment zone, such as a measure of part of the volume of the treatment zone when occupied by the first user, for example it can be a measure of the change in the volume of the treatment zone resulting from the presence of the first user.

In embodiments, the first operational parameter is determined directly from a function of the first volume indicator. In embodiments, the function is a numerical operation. In some embodiments, the function of the first volume indicator is simply the first volume indicator.

In embodiments, thermoregulation fluid is to be provided to the pack in accordance with the first operational parameter.

In embodiments, the thermoregulation zone is curved and the volume of the treatment zone is the volume within the curvature of the thermoregulation zone.

In embodiments, the thermoregulation zone surrounds the treatment zone.

In preferred embodiments, the thermoregulation zone is continuous around the treatment zone whereby to surround the treatment zone.

The first operational parameter can be: a first volume of thermoregulation fluid to be provided to the pack for treating the first user; a first flow rate of thermoregulation fluid to be provided to or from the pack; a first temperature of thermoregulation fluid to be provided to the pack; or a first pressure for pressurising the pack.

According to the invention, the first operational parameter is a volumetric parameter, preferably a first volume of thermoregulation fluid to be provided for treating the first user. Preferred embodiments of the invention are able to gain an indication of the volume occupied by a user, for example by a limb of the user, when the pack is placed upon the user in the position in which it will be used. This is advantageous because in embodiments, the size of the treatment zone is different for different users. Therefore, in many assemblies, the volume occupied by a user will affect the surface area of the thermoregulation zone which can provide thermal exchange with the user. This in turn will affect, for a given volume of thermoregulation fluid in the thermoregulation zone, the thickness of the layer of thermoregulation fluid for thermal exchange with the user. This in turn will affect the thermal transfer rate between the user and the thermoregulation fluid. Determining a first volume of thermoregulation fluid to be provided, from the first volume indicator, enables a volume of thermoregulation fluid to be provided in the thermoregulation zone that provides an appropriate thickness of thermoregulation fluid for the type of treatment and thermal transfer desired for the first user.

In some embodiments, the first volume of thermoregulation fluid is determined so as to provide the thermoregulation fluid in the thermoregulation zone with a desired thickness.

In embodiments, the first volume of thermoregulation fluid is a product of the first volume indicator and a ratio of a reference thermoregulation fluid volume to a reference volume indicator, wherein the reference volume indicator and the reference thermoregulation fluid volume are reference values associated with the type of pack to be used.

Some embodiments include:

obtaining data representing a type of pack to be used;
accessing a data repository containing a plurality of pack data sets, each pack data set including a reference volume indicator and a reference thermoregulation fluid volume for a different type of pack;
obtaining from the data repository the pack data set corresponding to the type of pack to be used.

In embodiments, the first operational parameter is determined in dependence on a treatment type.

In some embodiments, the method includes determining an algorithm for calculating the first operational parameter from the first volume indicator, and the step of determining, from the first volume indicator, the first operational parameter, includes calculating the first operational parameter using the first volume indicator and the algorithm.

Determining the algorithm can include obtaining algorithm data in dependence on a treatment type. Obtaining algorithm data can include obtaining the algorithm data from an algorithm data repository in dependence on a treatment type. For example, the method can include obtaining data relating to a treatment type, accessing an algorithm data repository, and obtaining algorithm data from the algorithm data repository in dependence on the data relating to the treatment type.

Further disclosed is a method of operating a personal thermoregulation system, the personal thermoregulation system including a personal thermoregulation pack with a thermoregulation zone, and a thermoregulation fluid supply system, the method including:

determining, in accordance with the above method, a first operational parameter for thermoregulation fluid to be provided for treating a first user;
operating the thermoregulation fluid supply system to supply thermoregulation fluid to the pack in accordance with the first operational parameter.

Preferably, the method includes operating the thermoregulation fluid supply to supply thermoregulation fluid to the pack whereby to provide the first volume of thermoregulation fluid.

Preferably, the pack includes a pressurising element for receiving pressure fluid and for applying pressure to the treatment zone, and obtaining the first volume indicator includes:

obtaining a target pressure fluid pressure and a reference pressure fluid volume, wherein the reference pressure fluid volume is a volume of pressure fluid that would cause the pressurising element to be at the target pressure fluid pressure with the treatment zone unoccupied; and
performing a first volume indicator determination procedure including:

operating the pressure fluid supply system to supply pressure fluid to the pressurising element with the first user occupying the treatment zone, whereby to increase the pressure of pressure fluid in the pressurising element;
detecting the pressure of pressure fluid in the pressurising element reaching the target pressure fluid pressure;
in response to detection of the pressure of pressure fluid in the pressurising element reaching the target pressure fluid pressure, determining a first volume of pressure fluid, the first volume of pressure fluid being a volume of pressure fluid that has caused the pressure of pressure fluid in the pressurising element to reach the target pressure fluid pressure;
determining a volume difference by subtracting the first volume of pressure fluid from the reference pressure fluid volume; and
determining the first volume indicator from the volume difference.

Preferably, determining the first volume of pressure fluid includes determining a volume of pressure fluid provided by the pressure fluid supply system.

In embodiments, the pressurising element is or includes a pressure chamber.

In embodiments, the first volume indicator is the volume difference.

In embodiments, the method can include draining the thermoregulation zone and/or the pressurising element before performing the first volume indicator determination procedure.

In embodiments, the pressurising element is able to expand substantially to fill the treatment zone when the treatment zone is unoccupied. Preferably, the treatment zone is substantially filled by the pressurising element when the treatment zone is unoccupied and the pressurising element is pressurised to the target pressure fluid pressure. The volume difference may in some embodiments equate to a volume of the first user for treatment.

The volume of the first user for treatment can be a first limb volume.

In embodiments, the reference pressure fluid volume is a volume of pressure fluid that would cause the pressurising element to be at the target pressure fluid pressure with the treatment zone unoccupied and with the pack in a first set of expansion conditions, wherein the first set of expansion conditions is a set of those conditions which have an effect on the relationship between the pressure and volume of pressure fluid in the pressurising element.

In embodiments, the reference pressure fluid volume is a volume of pressure fluid that would cause the pressurising element to be at the target pressure fluid pressure with the treatment zone unoccupied and with the thermoregulation zone empty.

Preferably, expansion of the pressurising element in all directions other than into the treatment zone is restricted.

In embodiments, when performing the first volume indicator determination procedure, the steps of:

operating the pressure fluid supply system to supply pressure fluid to the pressurising element with the first user occupying the treatment zone, whereby to increase the pressure of pressure fluid in the pressurising element;

detecting the pressure of pressure fluid in the pressurising element reaching the target pressure fluid pressure; and

in response to detection of the pressure of pressure fluid in the pressurising element reaching the target pressure fluid pressure, determining the first volume of pressure fluid;

are performed:

with substantially all fluid in the pack being pressure fluid; and/or

with the thermoregulation zone empty; and/or

with the pack in the first set of expansion conditions.

In embodiments, the pack is tubular and the treatment zone is radially inward with respect to the pressurising element.

In embodiments, the thermoregulation zone is curved and the pressurising element is configured to be able to apply pressure to the thermoregulation zone in a radially inward direction, for example by being disposed radially outwardly with respect to the thermoregulation zone.

In embodiments, the pack is tubular and the thermoregulation zone is disposed radially between the pressurising element and the treatment zone.

In some embodiments, the method can include monitoring the pressure of pressure fluid in the pressurising element and monitoring the volume of pressure fluid provided to the pressurising element.

In some embodiments, the reference pressure fluid volume can be a maximum pressure fluid volume for the pack, for example determined by the mechanical limits of the pack.

In some embodiments, obtaining a target pressure fluid pressure and a reference pressure fluid volume can include:

obtaining data representing a type of pack to be used;

accessing a data repository containing a plurality of pack data sets, each pack data set including a target pressure fluid pressure and a reference pressure fluid volume for a different type of pack;

obtaining from the data repository the pack data set corresponding to the type of pack to be used.

According to an aspect of the invention, there is provided a personal thermoregulation system for being coupled to, and providing a treatment to a user via, a personal thermoregulation pack which includes a thermoregulation zone for at least partially surrounding a treatment zone and for applying a thermal treatment to a user at the treatment zone, the thermoregulation system including an operation unit described below.

According to an aspect of the invention, there is provided an operation unit, the operation unit being for a personal thermoregulation system for being coupled to, and providing a treatment to a user via, a personal thermoregulation pack which includes a thermoregulation zone for at least partially surrounding a treatment zone and for applying a thermal treatment to a user at the treatment zone, the operation unit being operable to:

obtain a first volume indicator, the first volume indicator being indicative of a volume of a treatment zone of a first pack when occupied by a first user;

determine, from the first volume indicator, a first operational parameter for thermoregulation fluid to be provided for treating the first user with the first pack, characterized in that the first operational parameter is a volumetric parameter.

The first operational parameter can be: a first volume of thermoregulation fluid to be provided for treating the first user; a first flow rate of thermoregulation fluid to be provided to or from the first pack; or a first temperature of thermoregulation fluid to be provided to the first pack.

Preferably, the thermoregulation system includes a thermoregulation fluid supply system for supplying thermoregulation fluid to a coupled pack, and the operation unit is operable to operate the thermoregulation fluid supply system to supply thermoregulation fluid to a coupled pack in accordance with the first operational parameter, for example to provide the first volume of thermoregulation fluid.

It will be appreciated that providing the first volume of thermoregulation fluid can include not only initially supplying the first volume of thermoregulation fluid but also replenishing the first volume of thermoregulation fluid for example with replacement thermoregulation fluid from the thermoregulation fluid supply system. In some embodiments, the operation unit is operable to determine an algorithm for calculating the first operational parameter from the first volume indicator, and to determine, from the first volume indicator, the first operational parameter, by calculating the first operational parameter using the first volume indicator and the algorithm.

The operation unit can be operable to obtain algorithm data for determining the algorithm from an algorithm data repository in dependence on a treatment type. For example, the thermoregulation system can include a treatment type data obtaining element, which can for example be a user-operated input component, for obtaining data relating to a treatment type, and the operation unit can be operable to access an algorithm data repository and obtain algorithm data from the algorithm data repository in dependence on the data relating to the treatment type.

The algorithm data repository can be a database for example stored in a memory in the thermoregulation system.

The treatment type can be defined by an injury type.

The operation unit can include a processor and/or any other computing components suitable for performing its functions.

In embodiments, the thermoregulation system includes a pressure fluid supply system for supplying pressure fluid to a pressurising element of a coupled pack, a pressure obtaining element for obtaining a determination of a pressure of pressure fluid in a pressurising element of a coupled pack, and a pressure fluid volume determination obtaining element for obtaining a determination of a pressure fluid volume, wherein the operation unit is operable to:

 obtain a target pressure fluid pressure and a reference pressure fluid volume, wherein the reference pressure fluid volume is a volume of pressure fluid that would cause the pressurising element of the first pack to be at the target pressure fluid pressure with the treatment zone unoccupied; and
 perform a first volume indicator determination procedure, including:

  operating the pressure fluid supply system to supply pressure fluid to the pressurising element of a coupled pack, which is preferably the first pack and preferably has the first user occupying the treatment zone, whereby to increase the pressure of pressure fluid in the pressurising element of the coupled pack;
  obtaining via the pressure obtaining element a determination of a pressure of pressure fluid in the pressurising element of the coupled pack;
  detecting a pressure of pressure fluid in the pressurising element of the coupled pack reaching the target pressure fluid pressure;
  in response to detecting the pressure of pressure fluid in the pressurising element of the coupled pack reaching the target pressure fluid pressure, obtaining, via the pressure fluid volume determination obtaining element, a determination of a first volume of pressure fluid, the first volume of pressure fluid being a volume of pressure fluid that has caused the pressure of pressure fluid in the pressurising element of the coupled pack to reach the target pressure fluid pressure;
  determining a volume difference by subtracting the first volume of pressure fluid from the reference pressure fluid volume; and
  determining the first volume indicator from the volume difference.

Preferably, the pressure fluid volume determination obtaining element is operable to obtain a determination of a volume of pressure fluid provided by the pressure fluid supply system.

In embodiments, when performing the first volume indicator determination procedure, the operation unit is operable to:

 operate the pressure fluid supply system to supply pressure fluid to the pressurising element of a coupled pack whereby to increase the pressure of pressure fluid in the pressurising element of the coupled pack;
 obtain via the pressure obtaining element a determination of a pressure of pressure fluid in the pressurising element of the coupled pack;
 detect a pressure of pressure fluid in the pressurising element of the coupled pack reaching the target pressure fluid pressure; and
 in response to detecting the pressure of pressure fluid in the pressurising element of the coupled pack reaching the target pressure fluid pressure, obtain, via the pressure fluid volume determination obtaining element, a determination of the first volume of pressure fluid:

  with substantially all fluid in the coupled pack being pressure fluid; and/or
  with a thermoregulation zone of the coupled pack empty; and/or
  with the coupled pack in the first set of expansion conditions.

In some embodiments, the operation unit is operable to drain thermoregulation and/or pressure fluid from the coupled pack before performing the first volume indicator determination procedure.

The pressure obtaining element can in some embodiments be operable to monitor a pressure of pressure fluid in a coupled pack.

In some embodiments, the thermoregulation system includes a pack data obtaining element for obtaining data representing a type of pack to be used, and the operation unit is operable to:

access a data repository containing a plurality of pack data sets, each pack data set including a target pressure fluid pressure and a reference pressure fluid volume for a different type of pack;

obtain from the data repository the pack data set corresponding to the type of pack to be used.

In some embodiments, the thermoregulation system includes a pack data obtaining element for obtaining data representing a type of pack to be used, and the operation unit is operable to:

access a data repository containing a plurality of pack data sets, each pack data set including a reference volume indicator and a reference thermoregulation fluid volume for a different type of pack;

obtain from the data repository the pack data set corresponding to the type of pack to be used.

The data repository can be a database, for example stored in a memory in the thermoregulation system.

The pack data obtaining element can for example be a barcode reader or a user operated input component such as a touchscreen interface, a cursor-driven interface or one or more keys or buttons.

According to an aspect of the invention, there is provided a personal thermoregulation assembly, including:

a personal thermoregulation system as above; and a personal thermoregulation pack, for example the first pack, for being placed against a user and coupled to the personal thermoregulation system for providing a treatment to a user.

The pressure obtaining element can include a pressure sensor, disposed on the pack or in the thermoregulation system, configured to sense the pressure of pressure fluid in the pressurising element of the pack on which it is disposed or of a pack coupled to the thermoregulation system. For example, the pressure sensor can be disposed in an outlet path of the pressure fluid supply system.

The pressure fluid volume determination obtaining element can include a sensor, disposed on the pack or in the thermoregulation system, configured to sense the volume of pressure fluid. For example, the sensor can be disposed in an outlet path of the pressure fluid supply system and be configured to sense the volume of pressure fluid supplied by the pressure fluid supply system.

The operation unit is preferably operable to perform any one or more of the methods provided herein.

Preferably, the thermoregulation pack is for being placed entirely around a user's limb, for example is a cuff.

In embodiments, the thermoregulation zone includes at least one fluid path therein for the passage of thermoregulation fluid therethrough and a fluid inlet and a fluid outlet.

The thermoregulation pack can include an input for inputting thermoregulation fluid into the thermoregulation zone and an output for outputting thermoregulation fluid from the thermoregulation zone.

In embodiments, the thermoregulation system includes a thermoregulation fluid supply system including a supply system output attachable in fluid communication with a fluid inlet of a thermoregulation zone of a thermoregulation pack for supplying thermoregulation fluid thereto.

Preferably, the thermoregulation system includes:

an output port for coupling to an input of a personal thermoregulation pack;
a thermoregulation fluid supply system for supplying thermoregulation fluid to the output port;
an output pump for pumping thermoregulation fluid out through the output port; and
a pressure control system for selectively operating a pressurising element of a personal thermoregulation pack to apply a pressure independently of operation of the output pump.

In embodiments, the pressurising element of a pack extends fully over the thermoregulation zone of the pack.

By having a pressure control system which is controllable independently of the supply of thermoregulation fluid, the pressure control system is able to keep an even layer of thermoregulation fluid across a treatment region, a treatment region being an area of contact between a user and a pack over which thermal exchange between the user and the thermoregulation zone can occur, irrespective of the rate of supply of thermoregulation fluid. This can for example maintain the thickness of the thermoregulation zone reliably at a predetermined value, which can provide an appropriate level of thermal transfer between the pack and the user.

Preferably, during thermal treatment of a user, the pressure control system is configured, for example by the operation unit, to operate a pressurising element of a coupled pack at a lower pressure than thermoregulation fluid in the thermoregulation zone of the coupled pack.

Preferably, the thermoregulation system includes:

an input port for coupling to an output of a personal thermoregulation pack; and
an input pump for pumping thermoregulation fluid in through the input port;
wherein the pressure control system is for operating a pressurising element of a personal thermoregulation pack to apply a pressure independently of operation of the input pump.

It has been found that by having an input and output pump, the volume of fluid in a coupled pack can be precisely regulated. This can mean that the first volume of fluid can be reliably maintained in a pack. It can also prevent the pack ballooning by some fluid becoming trapped.

The operation unit can be operable to control the pumping rates of the input and output pumps independently of each other.

Preferably, the operation unit is operable to control, independently of each other, a pressure with which a coupled pack is pressed against a user, a rate flow of thermoregulation fluid out of the output port; a rate of flow of thermoregulation fluid in through the input port, and a temperature of thermoregulation fluid supplied through the output port.

References herein to the pressure with which a pack is pressed against a user or a patient can refer to either the total pressure with which the pack is pressed against the user or patient or to the pressure with which the pack is pressed against the user or patient by the pressurising element of the pack.

In some embodiments, the input pump is for pumping thermoregulation fluid in through the input port to the thermoregulation fluid supply system.

Preferably, pumping rates of the output and input pumps, and therefore the rate of thermoregulation fluid entering and leaving a pack coupled to the output and input ports, can be controlled independently of each other, meaning that in preferred embodiments the volume of fluid in a pack can be controlled precisely. The volume of thermoregulation fluid in a pack is typically the integral of the rate of thermoregulation fluid flow into the pack minus the integral of the rate of thermoregulation fluid flow out of the pack, meaning the volume in the pack can be reduced by pumping out at a greater rate than pumping in and the volume can be increased by pumping in at a greater rate than pumping out.

Additionally, in preferred embodiments, the volume of thermoregulation fluid in a pack can be controlled independently of the rate of thermoregulation fluid flowing into the pack. This is because the change in volume of the pack is dependent on the difference between the rates flowing in and out, and not on the absolute rates of flow.

In preferred embodiments, the pressure with which a coupled pack is pressed against a patient, the rate of flow of thermoregulation fluid into a coupled pack, the volume of thermoregulation fluid in a coupled pack, and the temperature of thermoregulation fluid entering a coupled pack can each be independently controlled, allowing the thermoregulation assembly to operate precisely to desired operation or feed parameters.

The rate of flow, the temperature, and other feed parameters of the thermoregulation fluid entering a coupled pack are also preferably the rate of flow, the temperature, and other feed parameters, of thermoregulation fluid out of the output port.

Having an input pump which actively pumps fluid out of the pack means that unwanted air can be pumped out of the pack to make way for thermoregulation fluid. Air is an insulator, meaning that it can interfere with energy transfer to and from the thermoregulation pack. Furthermore, air in the thermoregulation zone of the pack takes up space that could be occupied by thermoregulation fluid, thereby affecting the performance of the pack. In prior art packs, it was often necessary to have a restricted liquid return to function as an exhaust to allow air to escape from the pack. While a restricted liquid return can be used in embodiments of the invention, it is not necessary in all embodiments since the input pump can forcibly draw air out of the pack.

Preferably, the pressure control system is for selectively varying a pressure applied by a pressurising element of a personal thermoregulation pack independently of operation of the input and output pumps.

Preferably, the pressure control system includes a pressure fluid outlet port for coupling to a pressurising element of a personal interface pack and a pressure fluid pump for pumping pressure fluid into or out of the pressure fluid outlet port.

Preferably, the pressure fluid is air.

Preferably, the thermoregulation fluid is incompressible, preferably a liquid, preferably water.

Having a separate pressure control system means that in preferred embodiments the system can cause a pack to apply even treatment to an area of a patient while adjusting the energy provided to that area independently. It can also mean that only a thin layer of thermoregulation fluid needs to be provided adjacent to the patient by the pack as the pressure is provided by a separate element.

Preferably, the operation unit is operable to control the input and output pumps and the pressure control system. Preferably, the operation unit is operable to control the pressure fluid pump and any other controllable components.

Preferably, the thermoregulation system includes a heat control unit for providing thermoregulation fluid at a predetermined thermoregulation temperature.

In some embodiments, the heat control unit is operable, for example controlled by the operation unit, to control the temperature of provided thermoregulation fluid independently of the operation or rate of operation of the input and output pumps and independently of operation of the pressure control system.

Preferably, after determining the first volume of thermoregulation fluid, the operation unit is operable to operate the output pump and optionally the input pump to provide the first volume of thermoregulation fluid to a coupled pack before the pressure control system is operated.

In some embodiments, the heat control unit includes a temperature regulated fluid supply, and a mixing system. The regulated fluid supply can include a hot fluid supply for supplying hot fluid and a cold fluid supply for supplying cold fluid.

The mixing system can be operated by the operation unit.

In some embodiments, the mixing system is operable to circulate thermoregulation fluid from the temperature regulated fluid supply to the temperature regulated fluid supply without passing through a thermoregulation pack.

In some embodiments, the mixing system is operable to circulate thermoregulation fluid from the hot tank to the hot tank without passing through a thermoregulation tank and preferably without passing to or through the cold tank.

In some embodiments, the mixing system is operable to circulate thermoregulation fluid from the cold tank to the cold tank without passing through a thermoregulation tank and preferably without passing to or through the hot tank.

Preferably, the heat control unit includes a hot fluid supply and a cold fluid supply and a mixing system for mixing hot fluid from the hot fluid supply with cold fluid from the cold fluid supply to provide thermoregulation fluid at a predetermined thermoregulation temperature.

In some embodiments, the heat control unit includes a hot fluid supply for supplying hot fluid and a cold fluid supply for supplying cold fluid; and the control unit is operable selectively to operate the output pump to pump out through the output port thermoregulation fluid from the hot fluid supply, thermoregulation fluid from the cold fluid supply, or thermoregulation fluid including a mix of thermoregulation fluid from the hot and cold fluid supplies; and the control unit is operably selectively to operate the input pump to pump thermoregulation fluid in through the input port to the cold fluid supply, to the hot fluid supply, or to both the cold and hot fluid supplies.

Preferably, the mixing system includes a return feed for returned or recycled thermoregulation fluid, the mixing system being operable to mix fluid from the regulated fluid supply, such as hot fluid from the hot fluid supply and cold fluid from the cold fluid supply, with returned or recycled thermoregulation fluid, to provide thermoregulation fluid at a predetermined thermoregulation temperature.

In some embodiments, the return feed can be selectively coupled, for example by a first recycling or pack bypass conduit, to an output of the mixing system whereby to recycle or bypass thermoregulation fluid from, for example directly from, the mixing system to the return feed without passing through a thermoregulation pack. This can advantageously be used to bring a volume of thermoregulation fluid to a desired temperature before it is passed to a pack, preventing a patient being exposed to overly hot or overly cold temperatures.

In some embodiments, an input junction of a regulated fluid supply unit can be selectively coupled, for example by a recycling conduit, to an output junction of the regulated fluid supply unit, whereby to recycle thermoregulation fluid for example without passing through the regulated fluid supply.

In embodiments, the regulated fluid supply unit is a unit for outputting fluid from and inputting fluid to the regulated fluid supply and preferably includes the regulated fluid supply.

In some embodiments, an output junction of the regulated fluid supply unit, preferably of the hot fluid supply, can be selectively coupled, for example by a pack bypass conduit, to an input junction of the regulated fluid supply unit, preferably also of the hot fluid supply, whereby to bypass thermoregulation fluid from the said output junction of the regulated fluid supply unit to the said input junction of the regulated fluid supply unit without passing through a thermoregulation pack.

In some embodiments, the pack bypass conduit can serve as a recycling conduit to recycle thermoregulation fluid from a thermoregulation pack to a thermoregulation pack without passing through the regulated fluid supply.

A pack bypass pump can be provided coupled to the pack bypass conduit to pump thermoregulation fluid from an output junction of the regulated fluid supply unit to an input junction of the regulated fluid supply unit.

In some embodiments, a second recycling conduit is provided which can be selectively activated to recycle thermoregulation fluid from a thermoregulation pack to a thermoregulation pack without passing through the regulated fluid supply. A recycling pump can be coupled to the second recycling conduit to selectively pump thermoregulation fluid in a circulation that avoids the regulated fluid supply.

In some embodiments, the pack bypass pump can serve as the recycling pump for pumping thermoregulation fluid in a circulation that avoids the regulated fluid supply and the pack bypass conduit can serve as the second recycling conduit.

The thermoregulation system preferably includes a heat control unit bypass conduit which can be selectively activated to cause thermoregulation fluid entering the input port to be directed to the output port without passing through the heat control unit.

The thermoregulation system preferably includes a mixing system bypass conduit which can be selectively activated to cause thermoregulation fluid entering the input port to be directed to the output port without passing through the mixing system. This can advantageously be used to isolate fluid in a pack from the mixing system, for example when the mixing system is being operated to bring a volume of fluid to a desired temperature.

Selective coupling and selective activation of a conduit can be provided by a valve which is operable, for example under control of the control unit, to selectively direct incoming fluid to one or more of a plurality of output conduits.

Selective coupling can be provided by a conduit coupled to a selectively operable pump which is configured to prevent thermoregulation fluid flow through that conduit in a non-operating state and to pump thermoregulation fluid through that conduit in an operating state. Similarly, selective activation of a conduit can be provided by providing coupled to that conduit a selectively operable pump which is configured to prevent thermoregulation fluid flow through that conduit in a non-operating state and to pump thermoregulation fluid through that conduit in an operating state.

Couplings between components configured to direct or convey thermoregulation fluid can be provided by conduits configured to convey thermoregulation fluid therebetween.

A heat control unit bypass pump is preferably coupled to the heat control unit bypass conduit to pump or drive fluid through a thermoregulation pack and along the heat control unit bypass conduit. The heat control unit bypass pump can be reversible.

A mixing system bypass pump is preferably coupled to the mixing system bypass conduit to pump or drive fluid through a thermoregulation pack and along the mixing system bypass conduit. The mixing system bypass pump can be reversible.

In some embodiments the pack bypass conduit and/or the second recycling conduit, which may be the same conduit, can be selectively activated owing to the pack bypass pump and/or recycling pump, which may be the same pump, being a selectively operable pump which is configured to prevent thermoregulation fluid flow through the respective conduit in a non-operating state and to pump thermoregulation fluid through the respective conduit in an operating state. Furthermore, in these embodiments, the mixing system bypass conduit can be selectively activated by the mixing system bypass pump being a selectively operable pump which is configured to prevent thermoregulation fluid flow through the mixing system bypass conduit in a non-operating state and to pump thermoregulation fluid through the mixing system bypass conduit in an operating state. In these embodiments, the input and output pumps can be selectively operable pumps configured to prevent flow in a non-operating state, but other selective couplings or selective activations in the thermoregulation supply system are provided by valves.

The input and/or output pump can be provided in the mixing system.

The input pump can be directly coupled to an input of the mixing system.

The output pump can be directly coupled to an output of the mixing system.

An output of the input pump can be coupled, for example selectively coupled, to the heat control unit or to a regulated fluid supply unit to return thermoregulation fluid thereto.

A recycling conduit can be selectively coupled between an output of the input pump and an input of the output pump example in order to circulate thermoregulation fluid without passing it to the regulated fluid supply unit or in some embodiments without passing it to the heat control unit. This can be advantageous for example for maintaining thermoregulation fluid at a current temperature, or for circulating thermoregulation fluid within the thermoregulation system to bring it to a desired temperature before sending it to a pack.

The input pump and/or the output pump and/or the pack bypass pump can be reversible.

Where components are described as coupled, then unless they are described as directly coupled, other components may be coupled between them.

In some embodiments, the input, output and bypass pumps are connected to a variable valve configuration manifold which also means the system can send from one of two tanks (cold and hot) or a blend of both using one pump and then return to the pack via the second pump to the same or different tank via this manifold or indeed bypassing both tanks and simply circulate around the pack only using the third pump.

Preferably, each of the pumps of the thermoregulation system is selectively operable and independently controllable by the operation unit, for example, the operation unit can independently control a pumping rate of each pump.

According to an aspect of the invention, there is provided a kit or assembly including a thermoregulation system as above and at least one personal interface pack for coupling to the thermoregulation system.

Preferably, the kit includes a plurality of personal interface packs.

Preferably, the or each pack includes:

an input for coupling to the output port of the thermoregulation system;
an output for coupling to the input port of the thermoregulation system;
a thermoregulation zone for conveying thermoregulation fluid from the input to the output; and
a pressurising element for applying pressure to the thermoregulation zone to press the thermoregulation zone against a patient.

The thermoregulation zone can be or include a thermal chamber.

The method preferably includes operating a heat control unit, such as the heat control unit above, to provide thermoregulation fluid at a predetermined thermoregulation temperature.

In some embodiments, a pack tune sequence is performed in which:

1. Pack is filled with air to achieve a pre-defined pressure

2. Current Air Volume is recorded once target pressure is achieved

3. Max Air Volume (Pack Database) - Current Air Volume = Limb Volume

4. Limb Volume value acts as a modifier that adjusts Coolant Volume to optimise therapy for a specific user.

In such embodiments, Pack Database Values are pre-determined fixed values linked to a specific pack type and can include the following:

(a) Pack Target Air Pressure: This can be the grip pressure for a pack. This preferably remains the same for temperature-only profiles with no variation in the air compression. This would also preferably remain unchanged during the pack tuning cycle in order to calculate the limb volume. If a user has a bigger limb, this target pressure will be achieved with a lower volume of air than someone with a smaller limb.

(b) Max Air Volume: This is preferably the volume of air recorded when the target air pressure is achieved with no limb in a pack. This will preferably be calculated before for each pack type and stored within the system's software.

(c) Pack Coolant Volume: This is preferably the set dosing volume for a pack type. In the Pack Database, this can be a starting/datum value for the pack that is suitable for an average/datum limb. This can be dynamically controlled, but the database value is preferably a starting point that can then adjusted to once a value has been attained for a user's limb contact volume.

Embodiments include software databases for how to prime and run a pack for optimal performance and has a connected effect to efficiency of therapy. Based upon the efficiency of the pack, a unique and special database and algorithm can be provided that takes:

{Air in outer chamber
+ Pressure of air in outer chamber
+ Temperature of air in outer chamber
+ Volume of coolant in pack
= Surface area of coolant in contact of human
+ current average temperature across pack

+/= define flow rate of coolant + real time temperature + pressure target + pack database table}

According to an aspect of the invention, there is provided a program for performing any one or more of the above methods when executed on a computing device, such as an operation unit as described herein.

According to an aspect of the invention, there is provided a computing device programmed with the above program or otherwise configured to perform the steps of any one or more of the above methods. The computing device can be a operation unit of a thermoregulation system, for example the operation unit described herein.

Where the operation unit is described herein as operable to perform a particular function, it is to be appreciated that it can be programmed or otherwise configured to perform that function.

The operation unit can be a control unit of a thermoregulation system.

It is to be appreciated that certain embodiments of the invention as discussed herein may be incorporated as code (e.g., a software algorithm or program) residing in firmware and/or on computer useable medium having control logic for enabling execution on a computer system having a computer processor. Such a computer system typically includes memory storage configured to provide output from execution of the code which configures a processor in accordance with the execution. The code can be arranged as firmware or software, and can be organized as a set of modules such as discrete code modules, function calls, procedure calls or objects in an object-oriented programming environment. If implemented using modules, the code can comprise a single module or a plurality of modules that operate in cooperation with one another.

Brief Description of the drawings

Embodiments of the invention are described below, by way of example only, with reference to the accompanying drawings, in which:

Figure 1 is a schematic diagram of a thermoregulation assembly in accordance with an embodiment of the invention;

Figure 2 is a schematic side view of a thermoregulation pack in accordance with the embodiment of Figure 1;

Figure 3 is a cross-sectional view of thermoregulation pack of Figure 2;

Figure 4 is a schematic diagram of the thermoregulation assembly of the embodiment of Figures 1 to 3 showing a pack circulation operation;

Figure 5 is a schematic diagram of the thermoregulation assembly of the embodiment of Figures 1 to 4 showing an internal circulation operation;

Figure 6 is a schematic diagram of the thermoregulation assembly of the embodiment of Figures 1 to 5 showing pack and internal circulation operations simultaneously;

Figure 7 is a schematic diagram of the thermoregulation assembly of the embodiment of Figures 1 to 6 showing a supply operation;

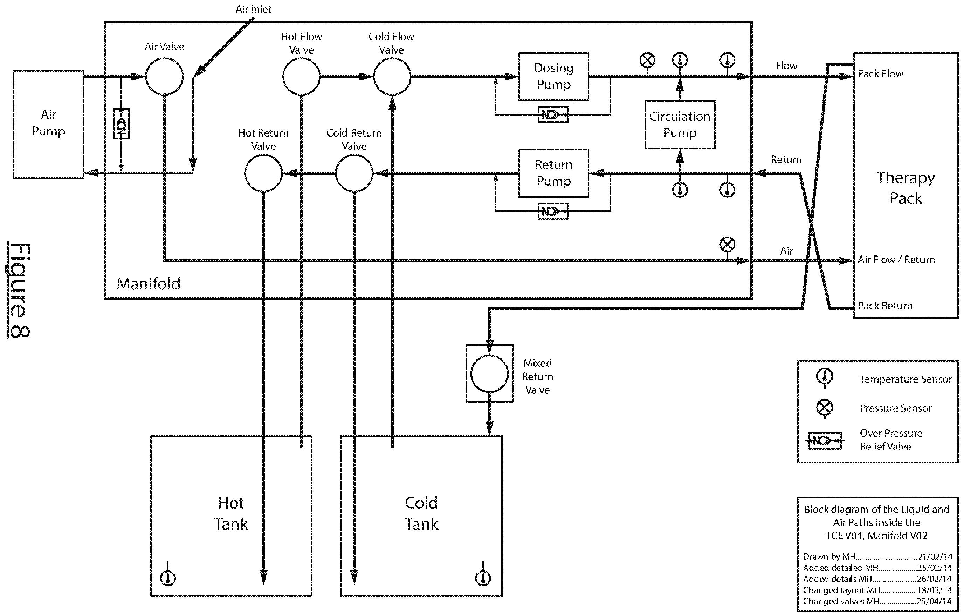

Figure 8 is a schematic diagram of a thermoregulation assembly according to an embodiment of the invention;

Figure 9 shows an isometric image of an alternative pack according to an embodiment of the invention;

Figure 10 shows various cross-sections of the pack of Figure 9;

Figure 11 is a schematic diagram of a thermoregulation assembly in accordance with an embodiment of the invention;

Figure 12 is a schematic diagram of the thermoregulation assembly of the embodiment of Figure 11 showing a pack circulation operation;

Figure 13 is a schematic diagram of the thermoregulation assembly of the embodiment of Figures 11 to 12 showing an internal circulation operation;

Figure 14 is a schematic diagram of the thermoregulation assembly of the embodiment of Figures 11 to 13 showing pack and internal circulation operations simultaneously;

Figure 15 is a schematic diagram of the thermoregulation assembly of the embodiment of Figures 11 to 14 showing a supply operation;

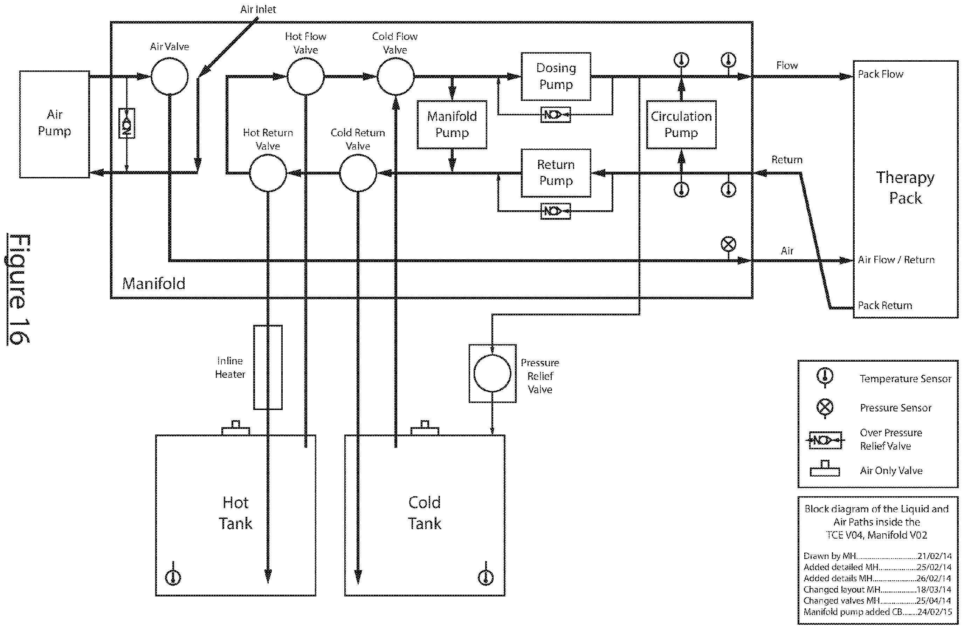

Figure 16 is a schematic diagram of a thermoregulation assembly according to an embodiment of the invention;

Figure 17 is a schematic diagram of the thermoregulation assembly of the embodiment of Figures 11 to 15 showing a hot tank internal circulation operation;

Figure 18 shows different pack configurations owing to two users having different limb sizes; and

Figure 19 shows steps in determining a user limb volume;

Figure 20 shows a limb volume in relation to a pack; and

Figure 21 is a table of algorithm data for an embodiment of the invention.

Detailed description of preferred embodiments

Embodiments of the invention relate to advantageous tuning methods for a thermoregulation assembly. However, before details of the methods are described in detail, preferred embodiments of a thermoregulation assembly with which the methods can be advantageously used are described.

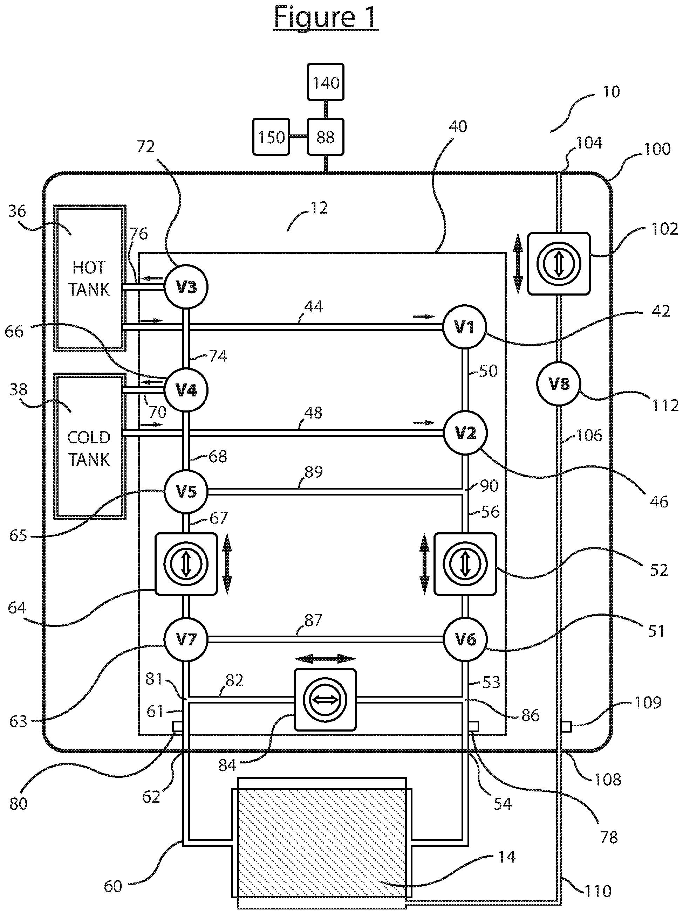

As can be seen from Figure 1, a thermoregulation assembly 10 includes a thermoregulation fluid supply system 12 and a thermoregulation pack 14.

The thermoregulation pack 14 is an interface pack for being placed around or against a part of a patient's body in order to apply a thermal treatment to alleviate or cure physical injuries or disorders.

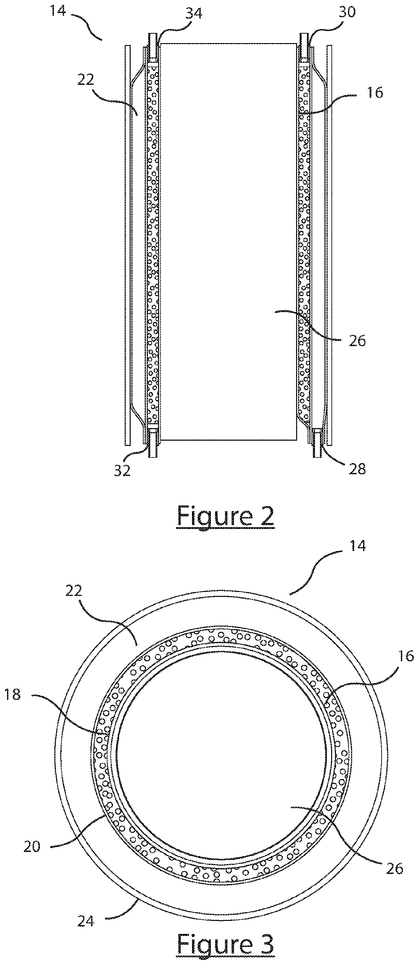

An example of a thermoregulation pack 14 is shown in Figures 2 and 3. In this example, the thermoregulation pack is a cylindrical cuff for example for going around a patient's limb.

In this embodiment, the cuff 14 includes a thermoregulation zone in the form of a thermal chamber 16 which is defined by the cylindrical space between a cylindrical inner wall 18 and a cylindrical separator wall 20. The distance between the cylindrical inner wall 18 and the cylindrical separator wall 20 is a thickness of the thermal chamber 16. A pressurising element in the form of a pressure chamber 22 is defined by the cylindrical space between separator wall 20 and outer wall 24, such that pressure chamber 22 completely surrounds thermal chamber 16 and is concentric with it. The hollow space defined by tubular inner wall 18 is the space into which a body part 26 is placed prior to treatment and can be considered to be a treatment zone. The cylindrical inner wall, to the extent that it forms a boundary between the treatment zone and the thermal chamber, forms a treatment region through which thermal treatment is applied to a user's limb by the thermal chamber.

In this embodiment, the inner and separator walls are displaceable, in other words can be moved radially, to allow for different sized limbs in the treatment zone and/or different pressurisations of the thermal and pressure chambers. However, in this embodiment, the outer wall 24 is configured to resist radial movement so that pressurisation of the pressure chamber causes the pressure chamber to expand predominantly radially inwardly.

In this embodiment, without a user's limb in the treatment zone, pressurisation of the pressure chamber can cause the pressure chamber to completely fill the treatment zone but maintain a substantially constant outer circumference.

In this embodiment, the thermal chamber 16 is filled with an open celled foam.

Figure 2 shows a side view of tubular cuff 14. Inner wall 18, separator wall 20 and outer wall 24 are formed from three tubes and these are bonded together at either end of the cuff 14 in order to terminate the thermal chamber 16 and the pressure chamber 22. The tubes may be hollow to enable fluid to be conveyed along them or alternatively may simply be formed by flexible solid material to function solely as a wall.

The thermal chamber 16 and the pressure chamber 22 are supplied with cooling/heating thermoregulation fluid and pressure fluid respectively through the ends of tubular cuff 14. The pressure fluid feed tube is connected to the pressure chamber 22 via a tubing port 28. A thermoregulation fluid feed is connected to the thermal chamber 16 via tubing port 30. A thermoregulation fluid return tube is connected to the thermal chamber 16 via tubing port 32. A restricted liquid return tube can be coupled to the thermal chamber 16 via tubing port 34.

Further details of the configuration of the thermoregulation pack 14 can be found in WO2013/190336 and WO2014/188213.

In general, different sized packs are provided for different sized users or for different limbs. In different sized packs, the outer wall is generally a different radial distance from the centre of the pack. In addition, a longitudinal length of the treatment region is different in different packs.

For reasons that will be evident from the description below, it is not necessary in all embodiments to provide tubing ports for a restricted liquid return. Furthermore, modifications described in WO2013/190336 can be applied to the pack 14 described herein, or other designs of pack can be used. However, for reasons which will become clearer below, the pack preferably has a pressure chamber which can be pressurised to cause the pressure chamber to expand to push against the thermal chamber towards the treatment zone. The pressure chamber is preferably restricted from expanding in directions other than towards the treatment zone. In a particularly preferred embodiment, the means for compressing comprises a chamber at least partially (and preferably completely) surrounding the thermal chamber and having an inlet and an outlet for a pressurising fluid, whereby putting said fluid under pressure in the second chamber compresses the thermal chamber into the body part.

Returning to Figure 1, the configuration of the thermoregulation fluid supply system 12 is described.

The thermoregulation fluid supply system 12 includes a hot fluid supply 36. In this embodiment the hot fluid supply is a hot tank of thermoregulation fluid. However, in other embodiments, the hot fluid supply 36 can be a port for receiving hot thermoregulation fluid from an external source. Typically, the thermoregulation fluid will be liquid. In the described embodiments the thermoregulation fluid is water, but other fluids especially liquids can be used.

The thermoregulation fluid supply system 12 also includes a cold fluid supply, which in this embodiment is a cold tank 38. However, in other embodiments, the cold fluid supply can be a port for coupling to an external source of cold thermoregulation fluid.

The hot and cold fluid supplies together form a temperature regulated fluid supply.

The hot tank 36 includes a heater and a temperature sensor and a feedback loop between the temperature sensor and the heater in order to maintain thermoregulation fluid in the hot tank at a predetermined temperature. Similarly, the cold tank 38 can include a cooler and a temperature sensor and a feedback loop between the temperature sensor and the cooler in order to maintain thermoregulation fluid in the cold tank at a predetermined temperature.

In other embodiments of the invention, the hot and cold tanks can be replaced by a single thermoregulation fluid supply which supplies fluid at a desired temperature. The thermoregulation fluid supply can include a single tank with a heater/cooler and a temperature sensor and can be operable to raise or lower the temperature of the thermoregulation fluid in the tank to a desired temperature.

The thermoregulation fluid supply system 12 includes a mixing system, in this case in a manifold 40, for mixing hot thermoregulation fluid from the hot tank with cold thermoregulation fluid from the cold tank and/or for mixing thermoregulation fluid from the regulated fluid supply with thermoregulation fluid from the pack 14.

The mixing system and manifold include a number of valves. Discussed below are two port valves and three port valves. Two port valves can be selectively positioned in an open or closed position. In the open position, the valve allows fluid to pass through it. In the closed position, the valve prevents the flow of fluid. The valves can be transitioned from the open to the closed state or vice versa rapidly in response to an electronic control signal. In other embodiments, the two port valves described below can be selectively placed into intermediate states, for example 50% open or 75% open, in order to allow different levels of flow through the valve. However, a valve which can be rapidly transitioned between the open and closed position by an electronic control signal is preferred since the effect of a partially open state can be obtained by rapidly opening and closing the valve.

Three port valves have either two input ports and one output port or two output ports and one input port. In some cases, where a port is not specified as input or output, it is because the role of the port can be interchanged during operation.

A three port valve with two input ports and one output port can be selectively positioned in 'input 1', 'input 2', 'input both' or 'closed' states. In the 'input 1' state, fluid from the first input port is allowed to flow to the output port, but fluid from the second input port is blocked. In the 'input 2' state, fluid from the second input port is allowed to flow to the output port, but fluid from the first input port is blocked. In the 'input both' state, fluid from both the first and second input ports is allowed to flow to the output port equally. In the 'closed' state, fluid is prevented from passing through the valve.

A three port valve with two output ports and one input port can be selectively positioned in 'output 1', 'output 2', 'output both' or 'closed' states. In the 'output 1' state, fluid from the input port is allowed to flow to the first output port but not the second output port. In the 'output 2' state, fluid from the input port is allowed to flow to the second output port, but not the first output port. In the 'output both' state, fluid from both the input port is allowed to flow to both the first and second output ports equally. In the 'closed' state, fluid is prevented from passing through the valve.

As for the two port valves, the three port valves can be transitioned between the four states rapidly in response to an electronic control signal, thereby effectively being able to produce combinations of the positions. However, in other embodiments, the three port valves can be operable to be positioned part way between two positions, for example to allow a 25% input from the first input and a 75% input from the second input.

The manifold 40 includes a two port hot tank flow valve 42, an input of which is coupled in fluid communication with the hot tank 36 via a conduit 44.

The hot tank flow valve 42 can be selectively positioned in an open or closed state.

The cold tank 38 is coupled in fluid communication with a cold tank flow valve 46 by a conduit 48. The hot tank flow valve 42 and the cold tank flow valve 46 are coupled in fluid communication by a conduit 50. The cold tank flow valve 46 is a three-port valve, with a first input coupled to the hot tank flow valve 42 and a second input coupled to the cold tank 38.

An output of the cold tank flow valve 46 is coupled in fluid communication via a feed or dosing pump 52 to a first port of a flow circulation valve 51 by a conduit 56. The flow circulation valve is a three port valve. A second port of the flow circulation valve 51 is coupled in fluid communication to an output port 54 of the fluid supply system 12 by a conduit 53.

In use, a tube 58 couples the output port 54 of the supply system 12 to a tubing port 30 on the pack 14.

The tubing port 30 is arranged so that in normal operation of the cuff 14 it is no lower than tubing port 32 in order that gravity facilitates the movement and return of fluid within the pack 14.

In use, tubing port 32 is coupled by a tube 60 to an input port 62 of the fluid supply system 12.

The input port 62 is coupled in fluid communication to a first port of a three port return circulation valve 63 by a conduit 61.

A second port of the return circulation valve 63 is coupled in fluid communication via a return pump 64 with a first port of a three port tank circulation valve 65 by a conduit 67.

A second port of the tank circulation valve 65 is coupled in fluid communication to a first input of a three port cold tank return valve 66 by a conduit 68.

A first output of the cold tank return valve 66 is coupled in fluid communication with the cold tank 38 by a conduit 70.

A second output of the cold tank return valve 66 is coupled in fluid communication with a two port hot tank return valve 72 via conduit 74. Hot tank return valve 72 is also coupled in fluid communication with the hot tank 36 via a conduit 76.

The system can include an inline heater, for example coupled to the conduit 76, for instantly heating fluid passing to the hot tank 36. The inline heater can be driven by fluid leaving the hot tank 36 by conduit 44 to ensure all fluid passing to the hot tank is as hot or hotter than fluid leaving the hot tank, thereby ensuring the hot tank maintains a fixed temperature.

Coupled to the conduit 53 between the flow circulation valve 51 and the output port 54 is a solid state safety cell 78. The solid state safety cell 78 includes a pressure sensor and a temperature sensor for measuring the temperature and pressure of thermoregulation fluid leaving the fluid supply system 12.

Coupled to the conduit 61 between the input port 62 and the return circulation valve 63 is a temperature sensor 80 for measuring the temperature of fluid passing through the input port 62.

Between the input port 62 and the return circulation valve 63, in particular in between temperature sensor 80 and the return circulation valve, is a junction 81 which couples a conduit 82 to the conduit 61. The conduit 82 can be considered to be a mixing system bypass conduit and couples the junction 81 to a junction 86 via a circulation or mixing system bypass pump 84. The junction 86 is coupled to the conduit 53 between the flow circulation valve 51 and the output port 54, in particular between the flow circulation valve 51 and the solid state safety cell 78.

The pumps 52, 64 and 84 can be reversible so as to be able selectively to pump fluid in either direction.

A third port of the return circulation valve 63 is coupled to a third port of the flow circulation valve 51 via a conduit 87 which can be considered a pack bypass conduit. A third port of the tank circulation valve 65 is coupled via a conduit 89 to conduit 56 at a junction 90 between the cold tank flow valve and the dosing or feed pump 52. The conduit 89 can be considered to be a recycling conduit.

There are no reservoirs in a fluid path from the feed pump 52 to the output port 54 or in a fluid path from the input port 62 to the return pump 64. This can mean that the pumps are able effectively to pump fluid into and out of a pack 14.

The thermoregulation fluid supply system 12 includes a control unit 88. Each of the temperature and pressure sensors described above is coupled to pass signals to the control unit 88. Furthermore, each of the heaters and coolers and pumps and valves described above is controllably coupled to the control unit 88 so that the control unit can operate them.

In the present embodiment, the mixing system can be considered to include the input and output pumps, the valves 42, 46, 65, 66 and 72, and the conduits 50, 56, 67, 68, 74 and 89. The manifold generally includes more components than just the mixing system. However, the precise extent of the mixing system is not important and could be considered to be any subset of the components of the manifold that serves to mix thermoregulation fluid so as to provide thermoregulation fluid at a desired temperature.

The hot and cold tanks together with the conduits 44, 48, 70 and 76, and the valves 42, 46, 66 and 72 can be considered to provide a regulated fluid supply unit, the valves 66 and 72 providing input junctions thereto and the valves 42 and 46 providing output junctions therefrom.

The thermoregulation fluid supply system, in particular the thermoregulation fluid pumps, are configured to supply or pump thermoregulation fluid at a fluid flow rate in the range from 200ml/min to 5000ml/min so that the thermoregulation fluid supply system can supply thermoregulation fluid to, or draw thermoregulation fluid from, the pack at this rate. However, this range can in other embodiments be different depending on the requirements of the assembly. In some embodiments, the range can be from 500ml/min to 2000ml/min. In some embodiments, the average fluid flow rate of thermoregulation fluid is about 1000ml/min.

The thermoregulation assembly 10 also includes a pressure control system including a pressure fluid supply system 100. The thermoregulation fluid supply system and the pressure control system can be said to provide a thermoregulation system. The pressure fluid supply system includes a pressure fluid pump 102 which can pump pressure fluid, typically air, from a pressure fluid source 104, typically the atmosphere, via a conduit 106 to a pressure fluid outlet 108. The pressure fluid outlet 108 can in use be coupled by a tube 110 to tubing port 28 of the pack 14. The pressure fluid supply system 100 can also include a pressure fluid valve 112.

The pressure fluid supply system also includes a pressure fluid sensor 109 for measuring the pressure of the pressure fluid in an attached pack and for measuring a volume of pressure fluid supplied via the outlet 108.

The pressure fluid pump 102 is reversible. The pressure fluid pump 102 and the pressure fluid valve are both coupled to the control unit 88 to be controllable thereby. The control unit is configured to operate the pressure fluid supply system so that the pressure of the pressure fluid in the pressure chamber is always at a lower pressure than the pressure of thermoregulation fluid in the thermal chamber. This can prevent the pressure chamber from causing blockages in the thermal chamber.

The thermoregulation assembly 10 can also include a port for receiving fluid from a liquid restricted return. However, since this is not necessarily in all embodiments, it is not described in detail herein.

Further options for the pressure fluid supply system and the liquid restricted return port can be found for example in WO2013/190336.

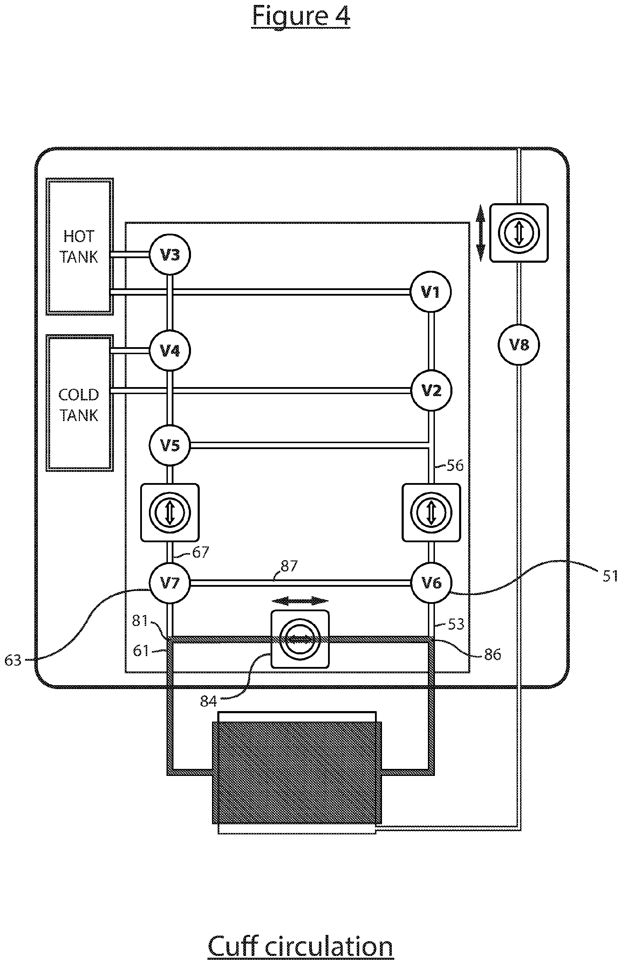

During operation, it is possible for thermoregulation fluid to be circulated around the pack 14 without passing into the mixing system or through the hot and cold tanks of the fluid supply system 12. This is shown in Figure 4. In order to do this, the return circulation valve 63 can be considered to have an input from conduit 61, and outputs to conduits 67 and 87. The return circulation valve 63 is operated in a "closed" state in order to block fluid from conduit 61.

Flow circulation valve 51 can be considered to have inputs from conduits 87 and 56 and an output to conduit 53. The flow circulation valve 51 is operated in a "input neither" state in which no fluid is passed to conduit 53.

The circulation or mixing system bypass pump 84 is operated to pump fluid in the direction of junction 86. Fluid, being unable to enter the flow circulation valve 51, passes through output port 54 and into pack 14. At the same time, the pump 84 is drawing fluid from junction 81, creating a negative pressure. As no fluid passes through the return circulation valve 63, this draws fluid from the pack 14 via the input port 62 and into the conduit 82. In this way, fluid is circulated through the pack 14 without passing through heat control apparatus. The circulation is carefully controlled because the pump 84 draws from the pack exactly what it provides to the pack. As there are no reservoirs between the pump 84 and the pack 14 in either direction, the positive pressure on one side of the pump 84 is able to drive fluid into the pack 14 and the negative pressure on the other side of the pump 84 is able to draw fluid out of the pack 14.

In some embodiments, the pump 84 can be operated in the opposite direction, which would be anticlockwise in Figure 4. However, since packs 14 typically have a preferred operating direction, this is not usually preferable.

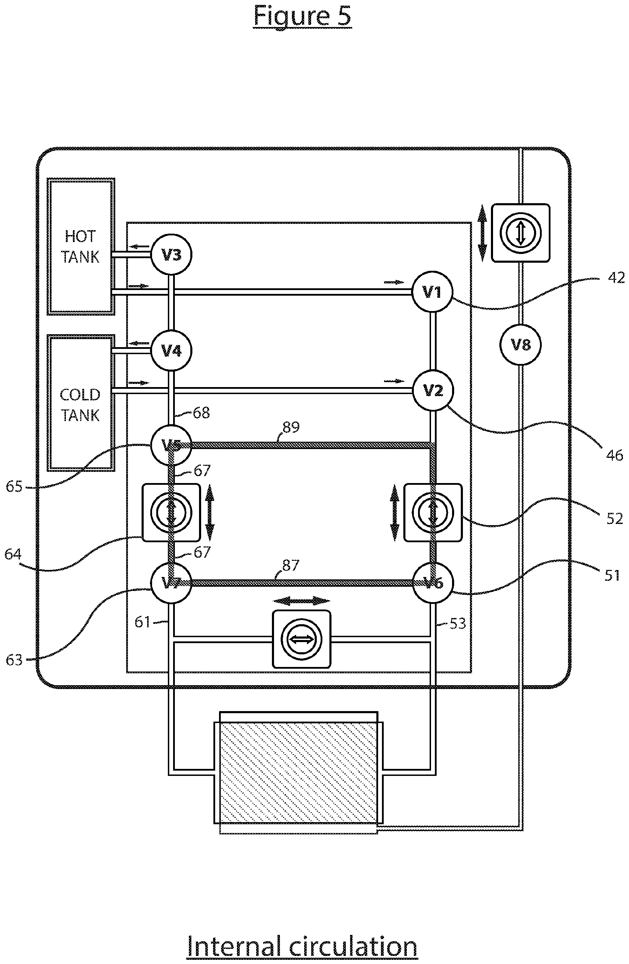

The assembly 10 can also be operated in an internal circulation mode, shown in Figure 5. In this situation, fluid is circulated within the fluid supply system 12 without passing to the pack 14. As can be seen from Figure 5, in this situation, the tank circulation valve 65 can be considered to have an input from conduit 67, a first output to conduit 68 and a second output to conduit 89. The return circulation valve 63 can be considered to have a first input from conduit 61, a second input from conduit 87, and an output to conduit 67. The flow circulation valve 51 can be considered to have an input from conduit 56, a first output to conduit 53, and a second output to conduit 87.

The tank circulation valve 65 is operated in a "output 2" mode, meaning that fluid from conduit 67 is passed only to conduit 89. Return circulation valve 63 is operated in a "input 2" mode meaning that only fluid from conduit 87 is passed to conduit 67. The flow circulation valve 51 is operated in a "output 2" mode meaning that fluid from conduit 56 is passed only to conduit 87. The return pump 64 is operated to pump fluid from return circulation valve 63 towards the tank circulation valve 65, and the dosing or feed pump 52 is operated to pump fluid from the tank circulation valve 65 towards the flow circulation valve 51. This causes fluid to continuously flow in a clockwise direction as shown in Figure 5.

The internal circulation shown in Figure 5 can be used advantageously to bring thermoregulation fluid to a desired temperature before passing it through the pack 14. In order to do this, the tank circulation valve 65 can rapidly be switched from "output 2" mode to "output 1" mode in order to pass thermoregulation fluid into conduit 68 and, by appropriate operation of the hot tank return valve and/or the cold tank return valve, this thermoregulation fluid can be returned to the hot or cold tanks. In addition, preferably simultaneously with the transition of the tank circulation valve 65 to the "output 1" mode, the hot tank flow valve and/or the cold tank flow valve 46 can be operated in order to allow a desired quantity of hot thermoregulation fluid from the hot tank and/or cold thermoregulation fluid from the cold tank to be drawn towards the dosing or feed pump 52 to join the thermoregulation fluid circulating. The tank circulation valve 65 is rapidly transitioned back to the "output 2" mode, and the cold tank flow valve 46 is transitioned into a "closed" mode. This can be repeated at intervals in order to gradually introduce hot and/or cold thermoregulation fluid to the circulating thermoregulation fluid in order slowly to bring the thermoregulation fluid to a desired temperature.

The internal circulation can be operated in an anti-clockwise manner by reversing the operation of the dosing or feed pump 52 and the return pump 64. However, this is not desirable since it makes it difficult to add hot and/or cold thermoregulation fluid from the hot and/or cold tanks.

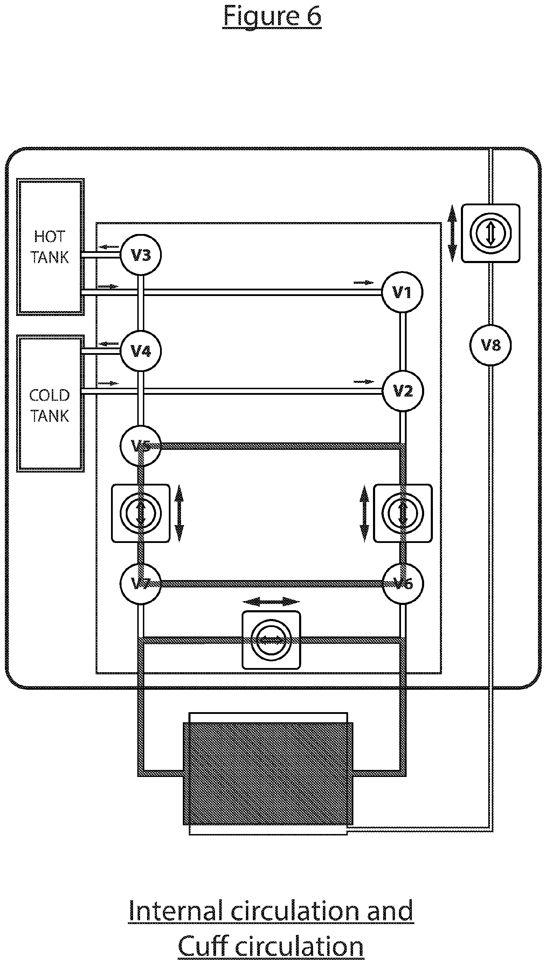

The internal circulation and the pack circulation can be operated simultaneously in order to circulate the existing pack fluid in the pack while further fluid is brought to the correct temperature. This is shown in Figure 6.

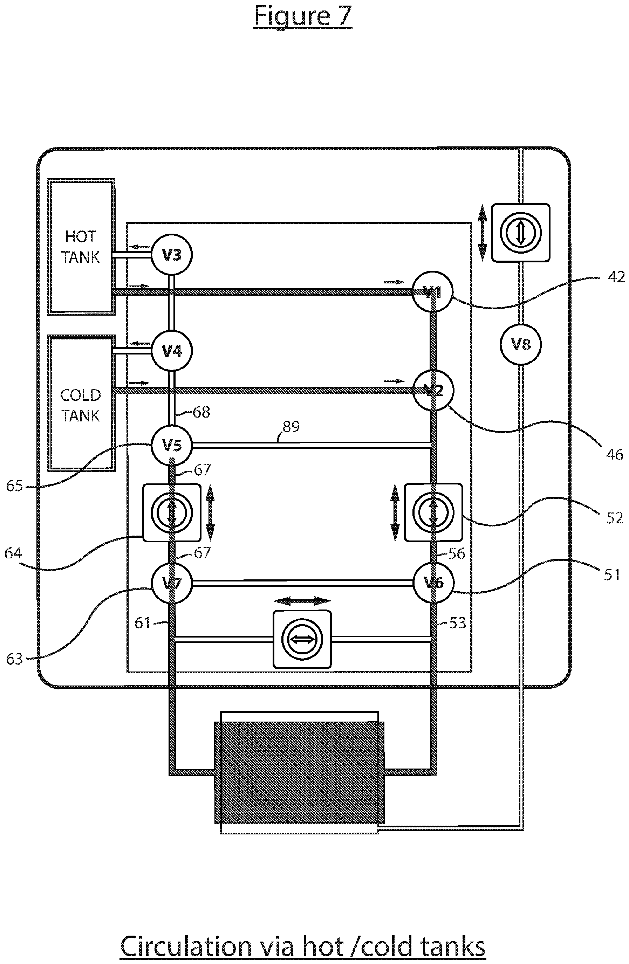

As shown in Figure 7, the assembly 10 can be operated to provide thermoregulation fluid directly to the pack 14 from the mixing system in particular from the hot and/or cold tanks. In this situation, the flow circulation valve 51 can be operated to allow fluid to pass only from conduit 56 to conduit 53; the return circulation valve can be operated to allow fluid to pass only from conduit 61 to conduit 67; and the tank circulation valve can be operated to allow fluid to pass only from conduit 67 to conduit 68. The hot and cold tank return valves can be operated as appropriate to allow fluid to pass into the hot and/or cold tanks.

The hot tank flow valve 42 and the cold tank flow valve 46 can be operated by the control unit 88 to transition between states in a rapid manner. These can therefore be operated by the control unit rapidly to switch between states in order to provide a desired ratio of hot fluid to cold fluid in order to provide thermoregulation fluid flowing into conduit 56 and then into conduit 53 and into the pack at a desired temperature in accordance with the desired feed parameters.

It is also possible for the tank circulation valve 65, instead of passing fluid from conduit 67 to conduit 68, to pass fluid from conduit 67 to conduit 89, and thereby recycle fluid within the mixing system, where it can optionally be mixed with fluid from the hot and/or cold tanks to adjust the temperature. In this mode of operation, the cold tank flow valve and hot tank flow valves are open for less than in the mode shown in Figure 7 since, unlike Figure 7, a large amount of the thermoregulation fluid to be supplied to the pack is recycled fluid from the pack. Preferably, the valves 42 and 46 only allow the addition of fluid from the hot and/or cold tank at intervals. As per the mode described in connection with Figure 5, when fluid is being added from the hot and/or cold tanks, fluid from conduit 67 can be allowed to return to the hot and/or cold tanks to avoid increasing the circulating volumes.

In both this mode of operation and the mode of operation shown in Figure 7, the feed or dosing pump 52 is operated to pump fluid into the pack at a predetermined rate, and the return pump 64 is operated to draw fluid out of the pack at a predetermined rate. As discussed elsewhere, the rates of the feed and return pumps can be equal if it is desired to maintain the existing volume of thermoregulation fluid in the pack, or they can be different if it is desired to change the volume of thermoregulation fluid in the pack.

In order to obtain thermoregulation fluid at a desired temperature, the control unit can operate the thermoregulation system to bring a volume of fluid to that desired temperature such as shown in Figures 5 and 6, or it can mix thermoregulation fluid as it is being fed to the pack as shown in and described for Figure 7.

While the thermoregulation system as discussed above can bypass the mixing system to keep a pack's thermoregulation fluid circulating around the pack without modification by the mixing system, and/or can bypass the pack to recycle thermoregulation fluid within or to the mixing system, particular advantages are realised when the mixing system is supplying thermoregulation fluid which is circulated through the pack using the feed and return pumps and the below relates to such a mode of operation. This may be after thermoregulation fluid has been brought to temperature using the configurations shown in Figures 5 and 6 and/or thermoregulation fluid may be being brought to temperature by the mixing system and circulated to the pack as part of the same cycle.

When thermoregulation fluid is to be supplied to the pack, the control unit operates the pressure fluid pump 102 to provide pressure fluid to the pack in accordance with operation or feed parameters, and operates the valves and pumps of the thermoregulation fluid supply system 12 to supply thermoregulation fluid in accordance with operation or feed parameters.

An advantage of having a feed pump 52 and a return pump 64 is that the volume and the flow rate of the thermoregulation fluid in the pack 14 can be accurately controlled. For example, beginning with the pack 14 empty, the control unit 88 can operate the feed pump 52 to pump thermoregulation fluid at a predetermined rate for a predetermined time into the pack 14. This will provide a known volume of fluid into the pack 14. At the end of this initial dosing, the pack 14 will contain a known volume of thermoregulation fluid.

From that point on, the feed pump 52 and return pump 64 can be operated at the same rate as each other. This will ensure that fluid is entering the pack 14 at the same rate as it is leaving the pack 14, thereby keeping the volume of the pack constant. While the prior art could ensure that thermoregulation fluid was entering the pack at a desired rate, in some positions or orientations, thermoregulation fluid could become trapped in the pack, and/or balloon in the pack and not return to the supply system at the same rate. This could cause a change in the volume of thermoregulation fluid in the pack and thereby affect the performance of the pack. Embodiments of the present invention by pumping into the pack at a known rate and pumping out of the pack at a known rate are able to ensure that the volume remains constant. The thermoregulation fluid is not able to become trapped or to balloon into the pack since it is being actively pumped out of the pack and it is not energetically favourable to remain in the pack.

It is mentioned above that it is not necessary in all embodiments to provide a liquid restricted return to the pack 14. This is because in embodiments of the present invention in which fluid is pumped into and out of the pack, all fluid, including air, is forcibly passed through the system and removed; it will not become trapped and interfere with the operation of the pack.

The operation of the feed pump 52 and the return pump 64 can be used to vary the flow rate of the thermoregulation fluid in the pack without varying the volume in the pack since, provided that the feed pump 52 and return pump 64 are being operated at the same rate, the rate can be increased or decreased as desired while maintaining the existing volume of thermoregulation fluid within the pack 14. Varying the flow rate in this manner varies the rate of thermoregulation fluid passing the patient and therefore the rate of energy being provided to or removed from the pack.

Alternatively, if it is desired to change the volume of thermoregulation fluid within the pack 14, the pumps can be operated at different rates. For example, if the feed pump 52 is operated at a higher rate than the return pump 64, the volume of the thermoregulation fluid in the pack 14 will increase at the rate of the difference between pumping rates. Correspondingly if it is desired to reduce the volume of thermoregulation fluid in the pack 14, the return pump 64 can be operated at a higher rate than the feed pump 52, and the volume in the pack 14 will decrease at the rate of the difference between the pumping rates. This can be useful if it is determined that the volume of thermoregulation fluid in the pack is causing too much energy to be transferred to or from the patient, risking burns, or too little energy to be transferred, making the pack inefficient.

While the pumps 52 and 64 are being operated to control the volume and the flow rate of the pack, and the pump 102 is being operated to control the pressure of the pack, the valves of the thermoregulation fluid supply system 12 can be operated as described above to adjust the temperature of the thermoregulation fluid being supplied to the pack.

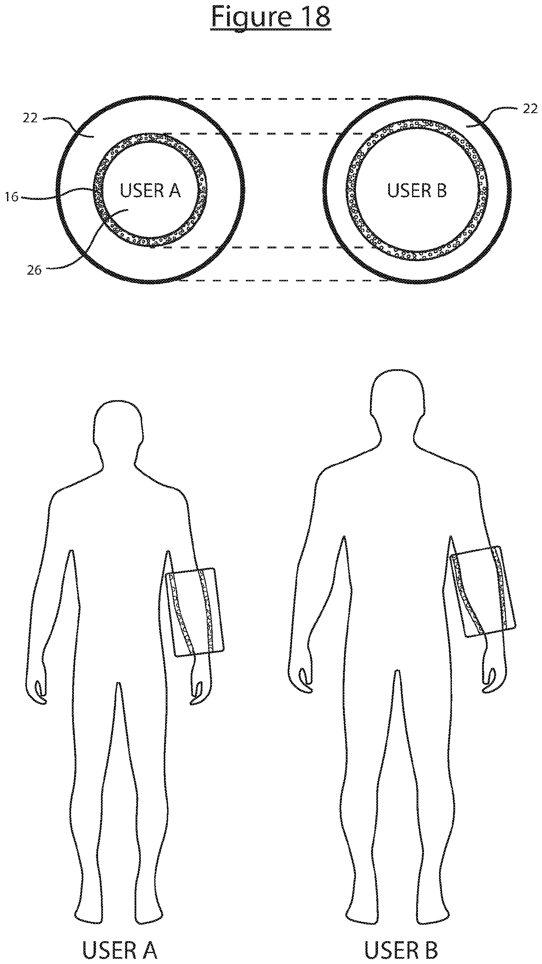

Having described the structure and operation of the thermoregulation assembly, it is described below how the assembly can be advantageously tuned with reference to Figures 18 to 21.

In the present embodiment, the control unit is configured to perform a pack tuning algorithm in order to determine a volume of thermoregulation fluid to be provided to a pack.

Embodiments of the invention are able to use a pack tuning algorithm to add a level of intelligence by considering the size of the user's limb and the pack type in use and determining therefrom an appropriate volume of thermoregulation fluid to use considering the treatment desired.

Any given pack may be used on different users, and the users' limbs are likely to have different sizes, shapes and have different thermal outputs depending on the user and the injury or treatment.

As can be seen from Figure 18, the area of the treatment zone is variable depending on the size of the user's limb. This means that users with larger limbs have a larger surface area in contact with the thermoregulation zone 26. Users with larger limbs can therefore be provided with an increased volume of thermoregulation fluid in order to cover the surface area of their limb to a desired thickness to ensure optimal performance and efficiency.

In order to perform a pack tuning algorithm, the control unit is configured to obtain a pack data set providing details of the thermoregulation pack being used.

In this embodiment, in order to obtain a pack data set, the control unit is configured to obtain data representing the type of pack to be used via a pack data obtaining element 140 coupled to or included in the control unit 88, and to access a database on a local or remote memory. The database includes a plurality of pack data sets, each set corresponding to a different pack type. Each pack data set includes predetermined fixed, reference, or starting values for a specific pack type. The control unit is configured to obtain the data set corresponding to the obtained data representing the type of pack to be used.

The pack data obtaining element can be any element for obtaining data relating to the type of pack to be used. In this embodiment it is a barcode reader, but it can be any other input component, such as a user-operated input component such as a touchscreen interface, a cursor-driven interface, or one or more keys or buttons.

In the present embodiment, each pack data set includes a pack target pressure fluid pressure representing a pressure of pressure fluid for the pressurising element of a pack of the associated pack type to provide a holding or grip pressure. Each pack data set also includes a reference thermoregulation fluid volume, which in this case is a dosing volume representing a starting volume of thermoregulation fluid for a pack of the associated pack type. The dosing volume is typically a preferred starting volume of thermoregulation fluid for a pack of the associated pack type to treat an average-sized limb. Each pack data set preferably also includes reference volume indicator, which is indicative of a volume of the average sized limb for which the dosing volume is suitable and in this embodiment is simply the volume of the average sized limb.

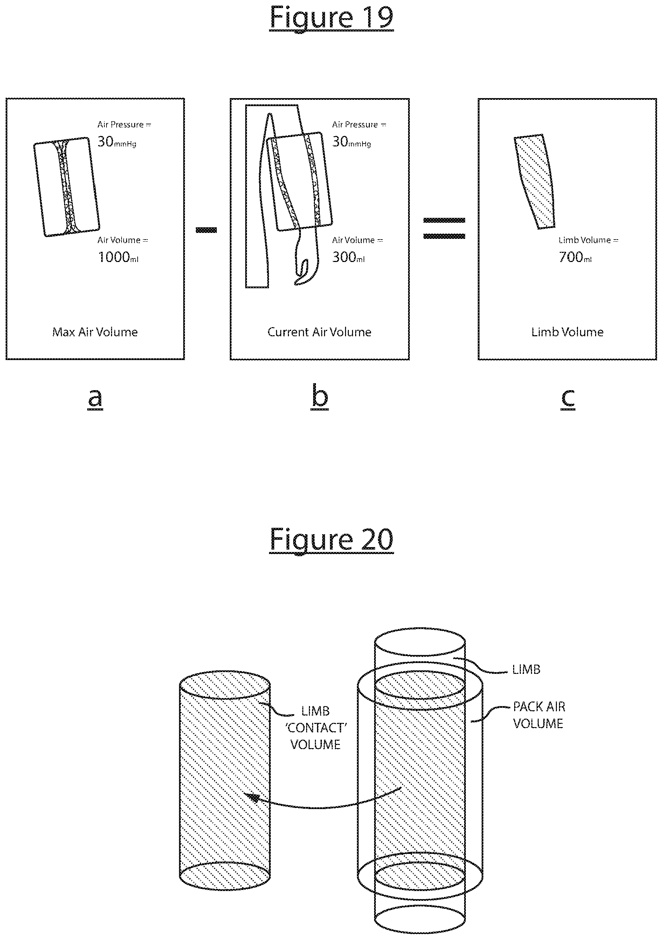

Each pack data set also includes a reference pressure fluid volume representing a volume of pressure fluid to be provided by the pressure fluid supply system that would cause a pressurising element of a pack of the associated pack type to be at the target pressure fluid pressure when the treatment zone is unoccupied. This can be determined in advance for a given pack type by measuring the volume of pressure fluid needed to pressurise the pressurising element to the target pressure fluid pressure when the treatment zone is unoccupied, as shown in Figure 19(a).

In the present embodiment, the reference pressure fluid volume is a maximum volume of pressure fluid that can be provided via the pressure fluid supply system when coupled to the pressurising element of a pack of the associated pack type, which in this embodiment is when the pressure chamber completely fills the treatment zone, which is the radially inner space in the cuff.

The control unit is configured then to measure a first volume indicator which is indicative of a volume of the treatment zone when occupied by a first user. In this embodiment, this is done by measuring a first user limb volume. Figure 20 shows the first user limb volume as compared to a thermoregulation pack.

In order to obtain a value for the first volume indicator, the control unit is configured, with the thermoregulation pack placed around the first user's limb and being substantially empty of thermoregulation fluid and pressure fluid, to operate the pressure fluid supply system to supply pressure fluid to the pressurising element of the thermoregulation pack up to the target pressure fluid pressure as provided in the pack data set.

While the control unit is operating the pressure fluid supply system to supply pressure fluid to the pack, the control unit is also configured to monitor readings from the pressure fluid sensor 109 to measure the volume of pressure fluid supplied as the pressure fluid in the pressurising element of the pack is being brought to the pack target pressure fluid pressure.

The control unit is configured to detect from the pressure fluid sensor 109 when the pressure fluid in the pressurising element of the thermoregulation pack has reached the predetermined pressure fluid pressure, and at that point to cease supplying further pressure fluid to the pack and to determine from readings from the pressure fluid sensor 109 the total volume of pressure fluid supplied, which is a first pressure fluid volume. If the user has a bigger limb, this target pressure will be achieved with a lower volume of pressure fluid than for a smaller limb. Figure 19(b) shows the situation when the first pressure fluid volume is determined.

The control unit is then configured to determine the first user limb volume from the first pressure fluid volume. By user limb volume what is meant is the volume of a user's limb to the extent that it is surrounded by the thermoregulation pack. This is unlikely to be the full volume of the entire limb.

The control unit is configured to determine the first user limb volume by subtracting the first pressure fluid volume from the reference pressure fluid volume from the pack data set. This is shown in Figure 19(c), and Figure 19 as a whole shows a schematic equation for obtaining the first user limb volume.

In some embodiments, for example in which the reference pressure fluid volume does not provide the maximum volume of pressure fluid when the pressure chamber completely fills the treatment zone, subtracting the first pressure fluid volume from the reference pressure fluid volume provides a volume difference which does not necessarily equal the first user limb volume, but which is still indicative of the first user limb volume as it differs from the first user limb volume by a reference volume of the treatment zone, the reference volume of the treatment zone being the volume of the treatment zone when the pressurising element of the pack is coupled to a pressure fluid supply system providing the reference pressure fluid volume at the reference pressure fluid pressure.

The control unit is configured to determine, from the first volume indicator, a first volume of thermoregulation fluid for the pack.

The control unit is configured to obtain, from a treatment type data obtaining element, data relating to a treatment type. The treatment type data obtaining element is in this embodiment a user interface 150 coupled to the control unit 88, but in other embodiments can be any other component for inputting data.

The treatment type data can include or be injury type data relating to an injury for which a particular treatment is suitable.

The control unit is operable to obtain from an algorithm database algorithm data in dependence on the data relating to the treatment type. The algorithm database may be stored in a memory in the control unit or remotely.

The algorithm data of the algorithm database can be used in an algorithm for converting the first volume indicator into the first volume of thermoregulation fluid. For example, if the treatment type data indicates that deep tissue needs to be targeted, the algorithm data will be used to convert a given volume indicator into a greater volume for thermoregulation fluid than if the treatment type data indicates that a light treatment is needed, such as a warm up or cool down.

By way of example, the algorithm data obtained from the algorithm database may include a treatment-dependent adjustment factor and the algorithm can be:

$$V = \frac{B}{A} \times F \times M$$

where:

B is the reference thermoregulation fluid volume from the pack data set;
A is the reference volume indicator from the pack data set;
F is the first volume indicator;
M is the treatment-dependent adjustment factor from the algorithm database; and
V is the first volume of thermoregulation fluid.

In some embodiments, the algorithm data obtained from the algorithm database includes multiple treatment-dependent adjustment factors for use at different stages of a treatment.

For example the algorithm data obtained from the algorithm database may include a deep tissue trauma multiple factor (H) and a post activity cool down multiple factor (I).

Such an embodiment can include several algorithms for different stages of treatment, for example:

$$G = \frac{B}{A} \times F$$

$$J = \frac{B}{A} \times F \times H;$$

and

$$K = \frac{B}{A} \times F \times I$$

where G is the first volume of thermoregulation fluid for a standard stage of treatment, J is the first volume of thermoregulation fluid for a deep tissue stage of treatment, and K is the first volume of thermoregulation fluid for a cool down stage of treatment.

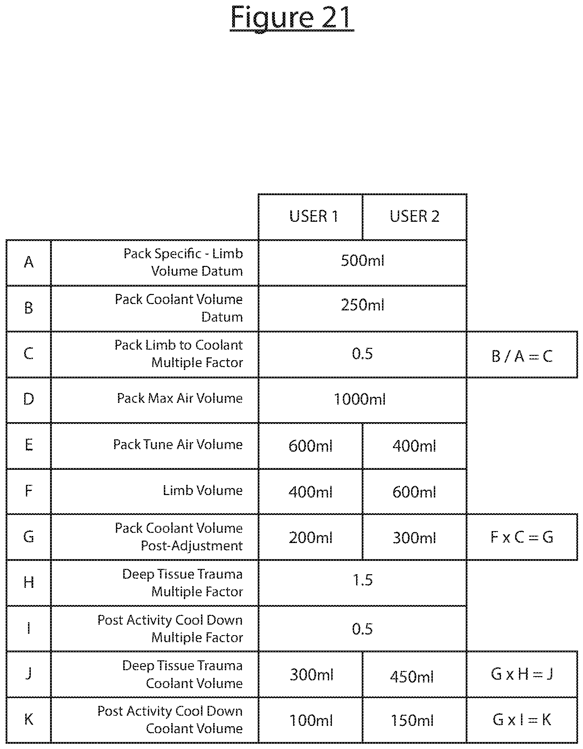

A specific example is shown in Figure 21.

Figure 21 shows the calculation of the first volume of thermoregulation fluid for two different users and for three stages of treatment. Although given slightly different names, the letters correspond to the letters mentioned above. In addition, D corresponds to the reference pressure fluid volume.

As can be seen from Figure 21, User 2 has a larger limb than User 1 and accordingly is provided with a greater volume of thermoregulation fluid at all treatment stages.

It is to be noted that although algorithm data has been described as being obtained from a database, some embodiments can operate without obtaining algorithm data, and can calculate the first volume of thermoregulation fluid as:

$$V = \frac{B}{A} \times F$$

Having determined the first volume of thermoregulation fluid, the control unit in some embodiments can be configured to obtain a set of operation or feed parameters in a similar manner to that described in WO2013/190336, with the constraint that the thermoregulation fluid volume is as calculated above. Although WO 2013/190336 describes a different thermoregulation and pressure system, the control system described therein, subject if necessary to any changes to account for the differences in the thermoregulation and pressure systems, can be applied to the present embodiment. However, the control unit does not always need to use the method disclosed in WO 2013/190336. In some embodiments, the control unit can use one or more of: the first volume indicator, the treatment type, a treatment temperature for example as input by a user, and any of the data in the pack data set, to determine feed or operation parameters. This can be for example from a look-up table.

In some embodiments, instead of or in addition to calculating the first volume of thermoregulation fluid to be provided, the control unit can be configured to determine one or more other feed or operation parameters from the first volume indicator and the data relating to a treatment type in order to compensate for the size of the user's limb and the treatment type. Other feed or operation parameters that may be determined include the temperature of the thermoregulation fluid to be supplied to the pack, the rate of flow of thermoregulation fluid to the pack or from the pack, and the pressure of the pressure fluid for the pack, to be applied to or to be in the pack. In some embodiments, the other feed or operation parameters can include the pressure of thermoregulation fluid for example in the pack or supplied to the pack. For example, deep tissue trauma with heavy bruising/muscle damage will have a high thermal output and will benefit from greater energy transfer. Furthermore, cold treatments benefit from a higher flow rate to remove energy quickly from the body; hot treatments benefit from a lower flow rate to put more energy into the body. In addition, larger limbs benefit from greater energy transfer than smaller limbs, and so the temperature difference from ambient can be increased for larger limbs, and/or the flow rate can be increased for larger limbs.

It is particularly advantageous to determine a volume of thermoregulation fluid to be used, when using the thermoregulation assemblies described herein, since the separate control of the pressure control system and the thermoregulation supply system means that the thickness of the thermoregulation zone of the pack can be more reliably controlled, and a calculated volume of thermoregulation fluid is therefore more likely to result in the desired thermal treatment.

Furthermore, in some embodiments it is possible to use the first pressure fluid volume as the first volume indicator without calculating the first user limb volume.

The tuning procedure for the preferred embodiment is shown in Figure 19.

As described above, in performing the pack tuning procedure, a user inputs to the control unit 88 the pack type, for example by scanning a barcode of the pack against the barcode reader 140 coupled to the control unit 88. The user also inputs a treatment type, for example by making a selection of possible injury types using the user interface 150.

The control unit then obtains from the pack database the associated pack data set.

The user can operate the control unit to drain the pack of thermoregulation and pressure fluid.

The user then places the thermoregulation pack substantially empty of thermoregulation fluid and pressure fluid around his or her limb and the control unit operates the pressure fluid supply system to supply pressure fluid to the thermoregulation pack up to the target pressure fluid pressure.

Once the pressure fluid in the pack has reached the target pressure fluid pressure, the control unit stops supplying further pressure fluid and determines the volume of pressure fluid required to obtain the target pressure fluid pressure.

The control unit then determines the first user limb volume by subtracting the volume of pressure fluid required to obtain the target pressure fluid pressure from the reference pressure fluid volume from the pack data set.

The control unit then obtains from the algorithm database algorithm data in dependence on the treatment type.

The control unit then calculates, in accordance with the above calculations, the first volume of thermoregulation fluid for the pack.

The control system may then determine other operation or feed parameters for operating the system as described in WO 2013/190336.

Having performed the tuning, the control system then operates the thermoregulation assembly in accordance with the operation or feed parameters, and uses the first volume of thermoregulation fluid for the pack.

It is to be noted that where more than one volume for thermoregulation fluid has been calculated, for example for different stages of treatment, the control system changes the volume of thermoregulation fluid as the stages of treatment change in accordance with the calculations.

It is also to be noted that once the user limb volume has been calculated, during further operation of the system, the pressure fluid pressure may be varied; it is not limited to the target pressure fluid pressure throughout operation. However, in some treatments, especially where there is temperature-only variation and not pressure variation, the pressure of the pressure fluid can remain at the target pressure fluid pressure. Furthermore, the target pressure fluid pressure can be used as a starting pressure fluid pressure.

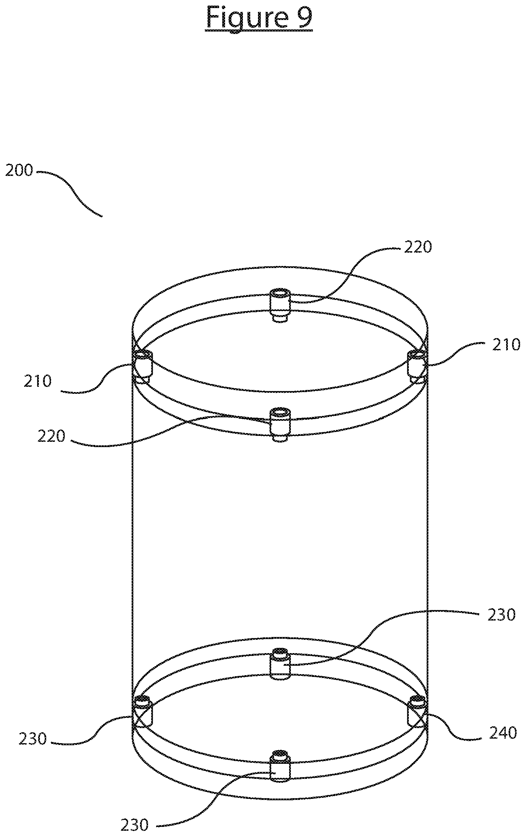

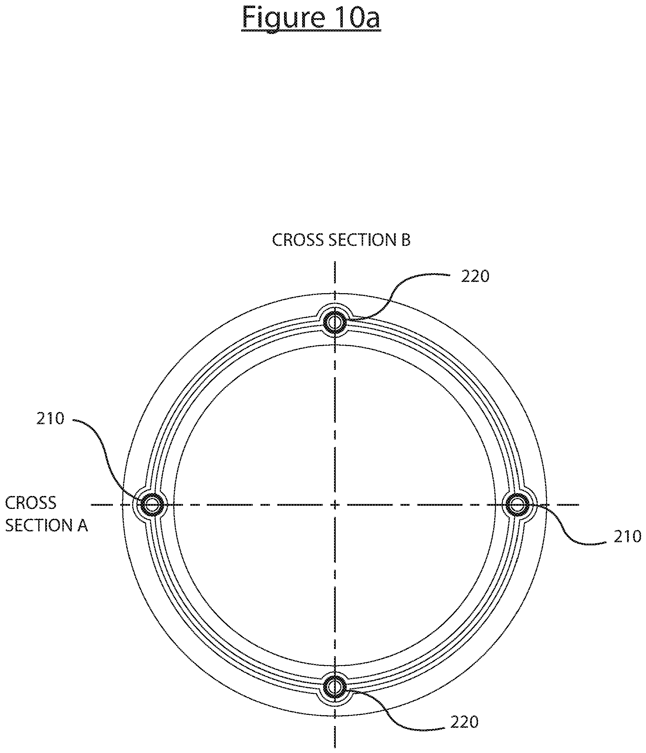

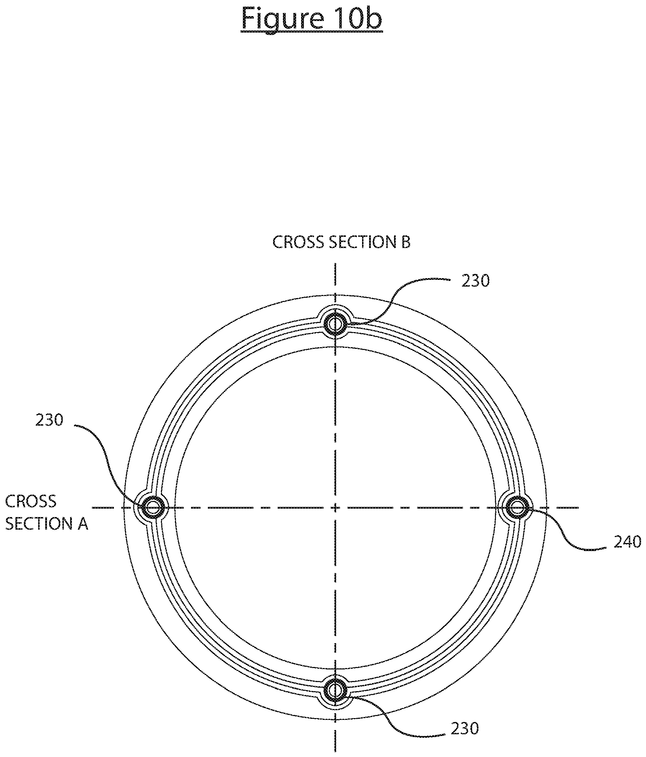

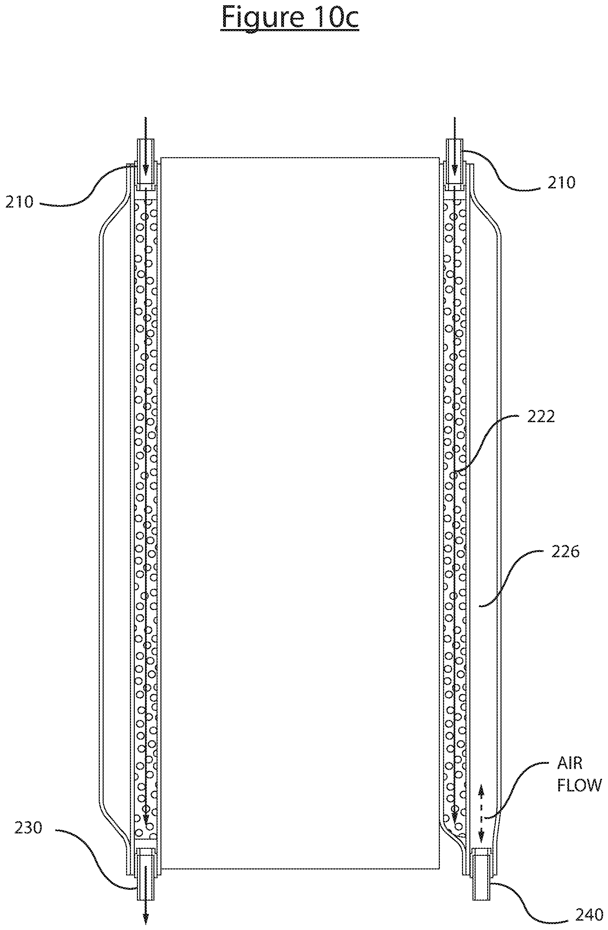

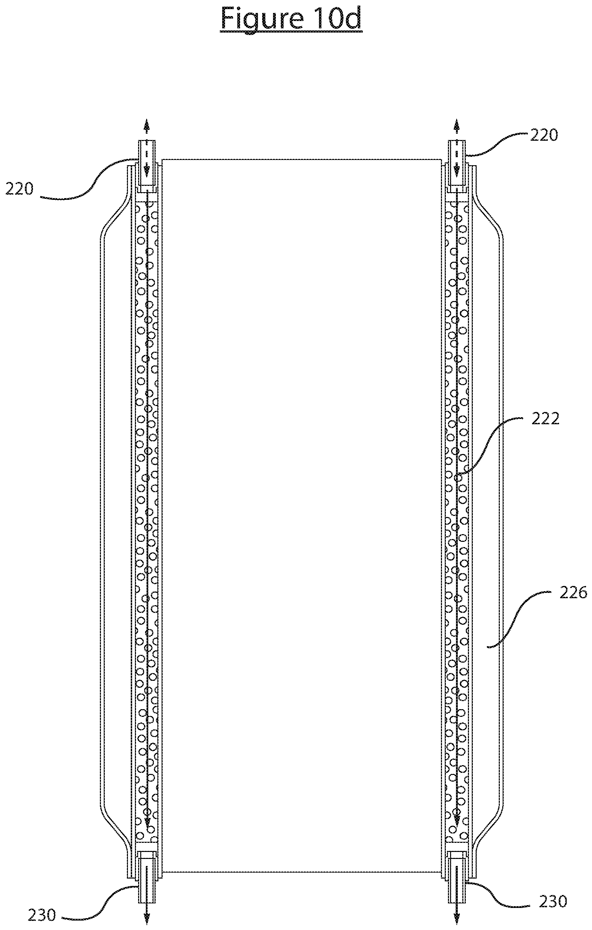

An alternative design of thermoregulation pack, in this case tubular cuff 200, is shown in Figures 9 and 10. Cuff 200 has 8 ports in total: 2 liquid feed and 2 restricted liquid return ports at the top, and 3 liquid return and 1 air feed at the bottom. Cuff 200 has thermal chamber 222 which is constructed as described for thermal chamber 16 above and pressure chamber 226 which is constructed as described for pressure chamber 22 above.

In more detail, Fig. 9 shows an isometric view of cuff 200 having two liquid feed ports 210 and two restricted return ports 220 at the top of cuff 200, and three liquid return ports 230 and one air feed port 240 at the bottom of cuff 200.

Cuff 200 is illustrated in more detail in top cross section in Fig. 10A and in bottom cross section in Fig. 10B. Fig. 10C then shows cross section view A and Fig. 10D shows cross section view B through Figures 10A and 10B.

In use, heating or cooling liquid is supplied to the cuff via liquid feed ports 210 and flows through the thermal chamber 222 as before, to exit via liquid return ports 230. Restricted return ports 220 function as an exhaust to allow any air to escape from the thermal chamber 222 as above. Pressure fluid is supplied to pressure chamber 226 as per the pack described above.

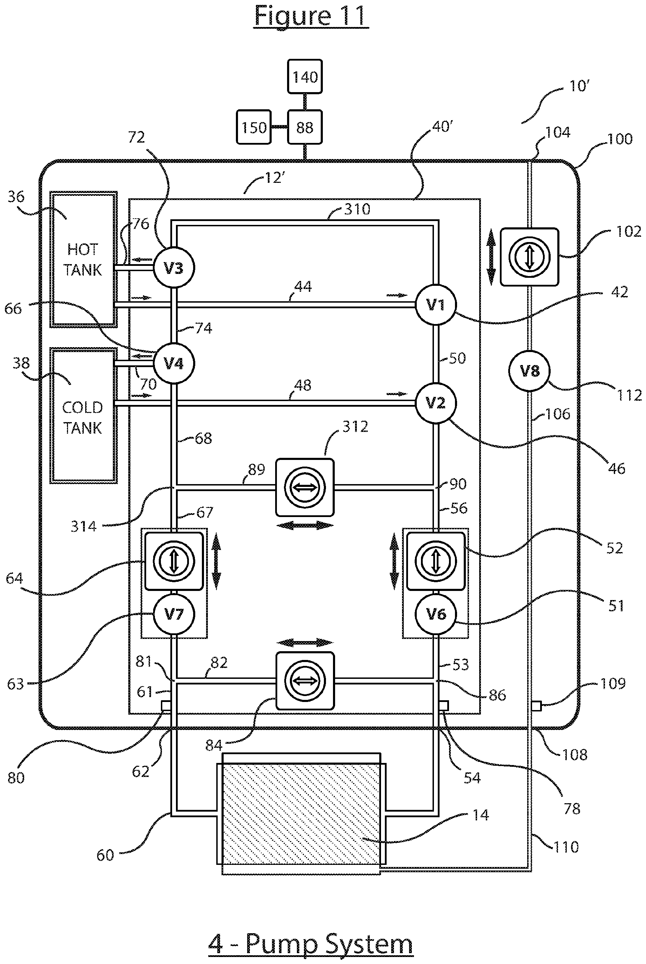

An assembly 10' according to another embodiment of the invention is shown in Figure 11. The features of the embodiment of Figure 11 correspond to the features of the embodiment of Figure 1 except for the differences outlined below. In particular, the pressure fluid supply system 100 is the same.

In the embodiment of Figure 11, the conduit 87 is omitted. Accordingly, the flow circulation valve 51 and the return circulation valve 63 can in this embodiment be two-port valves instead of three-port valves.

In addition, the hot tank return valve 72 and the hot tank flow valve 42 can be three-port valves instead of two-port valves, and the second output port of the hot tank return valve 72 can be coupled to the second input port of the hot tank flow valve 42 by a conduit 310. The conduit 310 can in this embodiment be considered to be a first recycling conduit as it can direct thermoregulation fluid from the hot tank return valve to the hot tank flow valve without passing through the hot tank itself.

In this embodiment, the hot tank 36, the cold tank 38, the hot tank return valve 72, the hot tank flow valve 42, the cold tank return valve 66, the cold tank flow valve 46, and the conduits 76, 44, 70 and 48 can be considered to provide a regulated fluid supply unit in which the hot tank return valve, and the cold tank return valve 66 provide input junctions thereto, and the hot tank flow valve 42, and the cold tank flow valve 46 provide output junctions therefrom. As described above, by operation of the valves 72, 66, 42, 46, thermoregulation fluid can be selectively directed into the hot and cold tanks from the regulated fluid supply unit input junctions, and out of the hot and cold tanks by the regulated fluid supply unit output junctions or can be directed past the hot and cold tanks. The first recycling conduit 310 is able to direct fluid from the regulated fluid supply unit input junction 72 to the regulated fluid supply unit output junction 42 whereby to recycle thermoregulation fluid within the thermoregulation fluid supply system. This can be advantageous where fluid is being circulated in the thermoregulation fluid supply system 12' without being passed to the hot or cold tanks.

In addition, in this embodiment, the tank circulation valve 65 is omitted and the conduit 89 includes a pack bypass pump 312 coupled to it. Accordingly, in this embodiment, the conduit 89 is coupled to a junction 314 between the conduit 67 and 68 instead of being coupled to the tank circulation valve 65.

The pack bypass pump 312 is configured to prevent fluid flowing through the pack bypass pump 312 along conduit 89 except when the pump 312 is being operated to pump fluid therealong. In other words, when the pack bypass pump 312 is not operating, it blocks fluid from travelling along the conduit 89. In the preferred embodiment, this function is provided by the pack bypass pump being a geared pump. In this embodiment, the pack bypass pump 312 is reversible so as to be able to pump fluid in either direction along the conduit 89.

In some embodiments, in a similar manner to the pack bypass pump 312, the return pump and/or the feed pump can be configured so as to prevent fluid from passing through them except when they are operating to pump fluid. For example, like the pack bypass pump 312, they can be geared pumps. This can mean that in some embodiments, the return circulation valve 63 and the flow circulation valve 51 can be omitted altogether.

In some embodiments, the mixing system bypass pump 84 can be configured to prevent fluid from passing along the conduit 82 except when the mixing system bypass pump 84 is pumping fluid therealong. For example, it can be a geared pump.

In the present embodiment, the mixing system can be considered to include the input and output pumps, the valves 42, 46, 66 and 72, the pump 312, and the conduits 50, 56, 67, 68, 74, 89 and 310. However, as described above, the precise extent of the mixing system is not important.

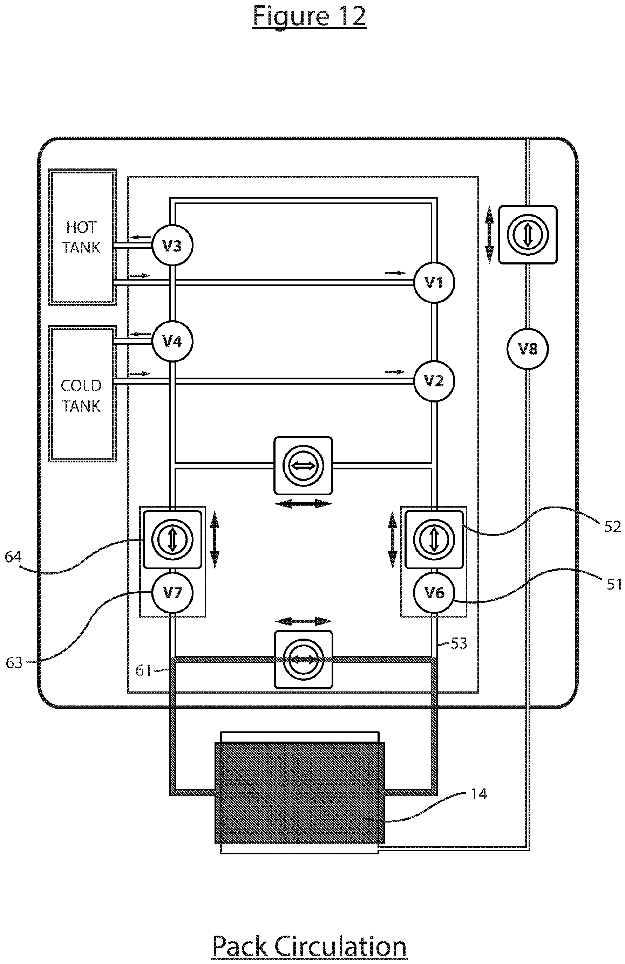

During operation of the embodiment of Figure 11, as for the embodiment of Figure 1, it is possible for thermoregulation fluid to be circulated around the pack 14 without passing into the regulated fluid supply. This is shown in Figure 12 and operates in a similar manner to that shown and described with respect to Figure 4. However, in embodiments in which the return circulation valve 63 and the flow circulation valve 51 are omitted, thermoregulation fluid can be obstructed in conduits 61 and 53 by the pumps 64 and 52 being in a non-operating configuration.

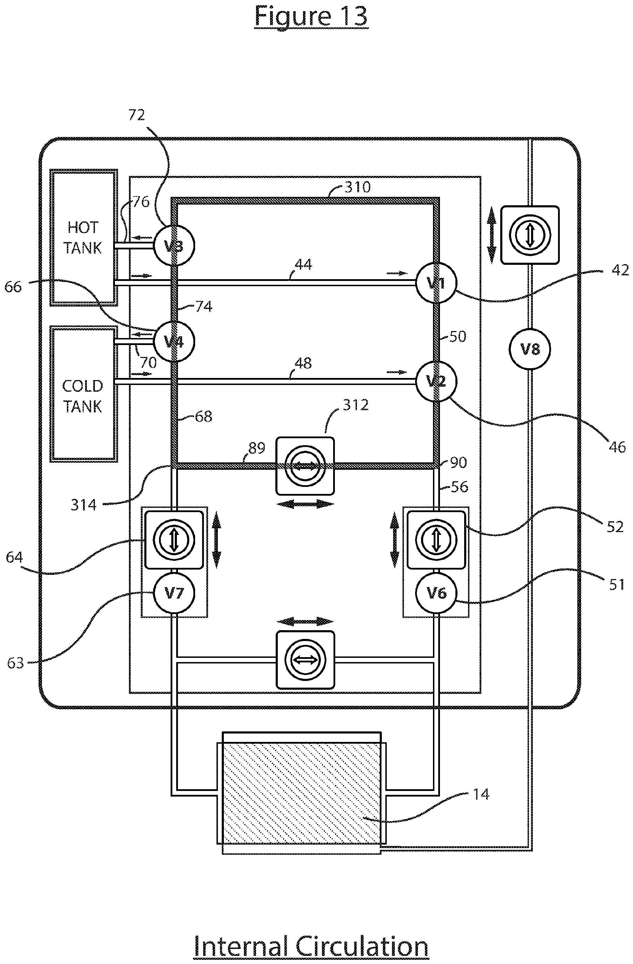

The assembly 10' can also be operated in an internal circulation mode as shown in Figure 13. As for the mode of operation shown in Figure 5, in this situation, fluid is circulated within the mixing system without passing to the pack 14. In this embodiment, the dosing or feed pump 52 and return pump 64 are not operating. In the embodiments in which the flow circulation valve 51 and the return circulation valve 63 are present, these are in the closed position. Accordingly, fluid does generally not flow closer to the pack 14 than the second recycling conduit 89.

The dosing or flow pump 52 and the return pump 64 do not need to be operated in this embodiment since the internal circulation is provided by the pack bypass pump 312. While this can be operated in either direction, for internal circulation it is preferred to operate the pump 312 to pump fluid in the direction from junction 90 to junction 314 as this makes it easier to add and remove fluid from the hot and/or cold tanks.

Because of the presence of the first recycling conduit 310, thermoregulation fluid can pass from the pump 312 to junction 314, along conduit 68, 74, 310 and 50.

Fluid can then pass to the junction 90 and return to the pump 312 along conduit 89. In order for this to be achieved, the cold tank return valve 66 is configured to allow fluid from conduit 68 to pass to conduit 74 but not conduit 70. The hot tank return valve 72 is configured to allow fluid from conduit 74 to pass to conduit 310 but not to conduit 76. The hot tank flow valve 42 is configured to allow fluid from conduit 310 to pass to conduit 50 but not to accept fluid from conduit 44. The cold tank flow valve 46 is configured to allow fluid from conduit 50 to pass to conduit 56 but not to accept fluid from conduit 48.

As for the internal circulation of Figure 5, the internal circulation of Figure 13 can be used advantageously to bring thermoregulation fluid to a desired temperature. This can be performed by appropriate operation of the hot and cold tank flow and return valves to allow thermoregulation fluid to be passed to the hot and/or cold tanks and/or to allow thermoregulation fluid from the hot and/or cold tanks to be added to the circulating thermoregulation fluid. This can be repeated at intervals in order gradually to introduce hot and/or cold thermoregulation fluid to the circulating thermoregulation fluid in order slowly to bring the thermoregulation fluid to a desired temperature.

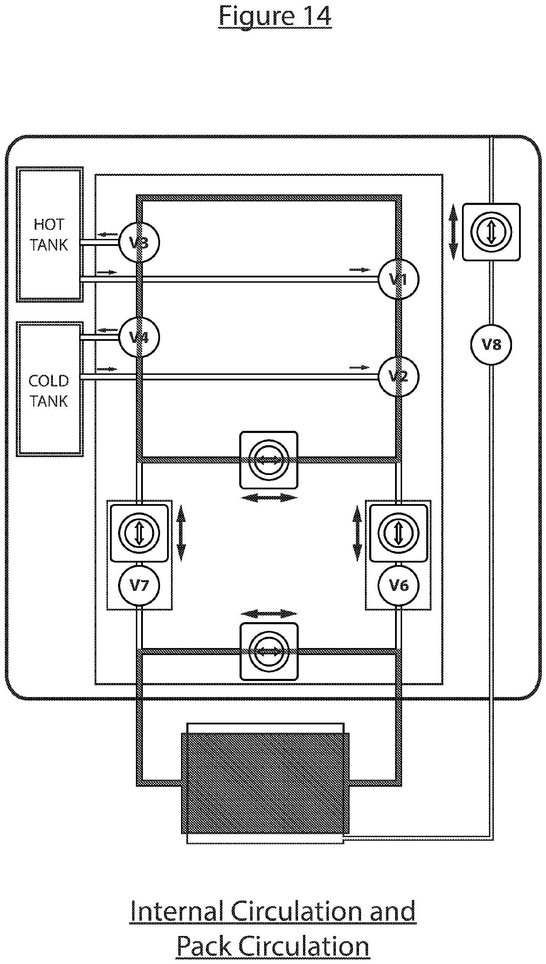

The internal circulation and the pack circulation can be operated simultaneously in order to circulate the existing pack fluid while further fluid is brought to the correct temperature. This is shown in Figure 14 and shows the simultaneous operation of the operations of Figures 12 and 13.

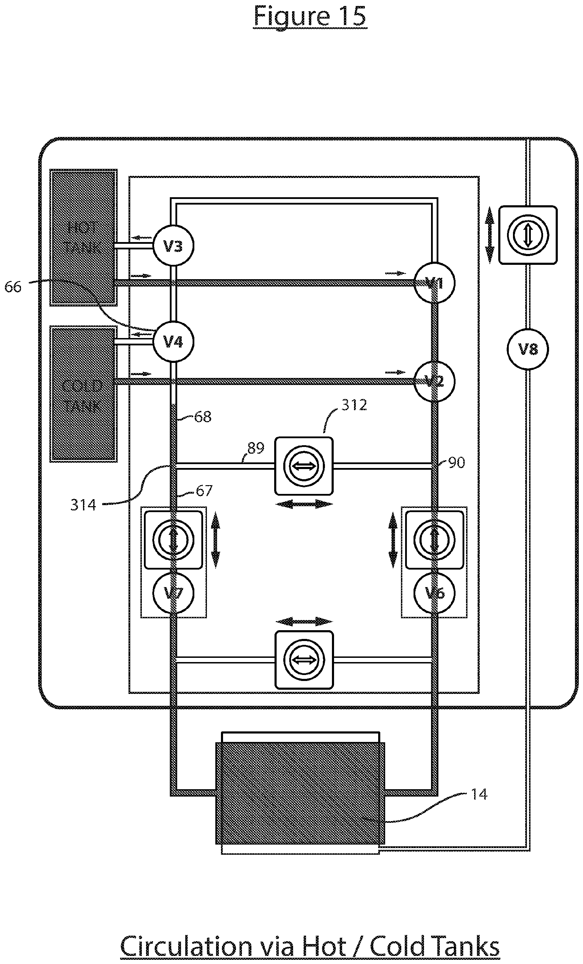

Figure 15 shows the assembly 10' being operated to provide thermoregulation fluid directly to the pack from the mixing system, in particular from the hot and/or cold tanks. This method of operation is similar to that described in respect of Figure 7. However, in this embodiment, there is no tank circulation valve 65. Thermoregulation fluid is prevented from passing to junction 90 from junction 314 by the pack bypass pump 312 being in a non-operating position and thereby blocking the passage of fluid along conduit 89. The thermoregulation fluid is free to flow from conduit 67 into conduit 68.

Furthermore, in order to recycle fluid without passing it to the hot and/or cold tanks, it is possible to transition the cold tank return valve 66 into a fully closed configuration and to operate the pack bypass pump 312 to pump fluid in the direction from junction 314 to junction 90 thereby serving as a recycling pump. In this way, fluid from the pack 14 is passed from junction 314 to junction 90 to be recycled without passing to the regulated fluid supply unit. However, at intervals, the operation can be transitioned to that described above in respect of Figure 15 in order to remove some thermoregulation fluid from the circulating fluid and/or to add some thermoregulation fluid from the regulated fluid supply in order to change the temperature of the thermoregulation fluid being supplied to the pack.

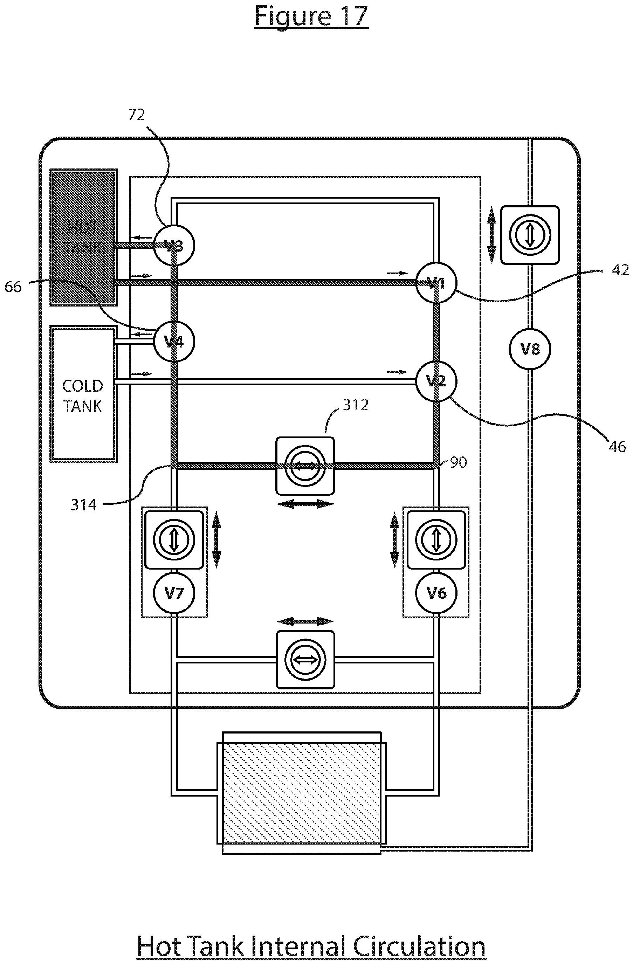

As shown in Figure 17, it is also possible by appropriate operation of the valves 42, 46, 66 and 72, and by operating the pack bypass pump 312 to pump fluid from junction 90 to junction 314 to operate a hot tank internal circulation.

This allows the thermoregulation fluid in the hot tank to be mixed as the thermoregulation fluid supply system prepares before treatment. This prevents an inconsistent temperature in the hot tank, prevents a temperature gradient occurring and enables the hot tank to get to temperature more efficiently and deliver a more accurate temperature of thermoregulation fluid.

A corresponding cold tank internal circulation is also possible.

In embodiments, the hot tank and cold tank can be interchanged.

## Claims

1. A method of determining an operational parameter for thermoregulation fluid to be provided for a personal thermoregulation treatment pack (14, 200), the pack including a thermoregulation zone for at least partially surrounding a treatment zone (26) and for applying a thermal treatment to a user at the treatment zone (26), the method including:

measuring a first volume indicator, the first volume indicator being indicative of a volume of the treatment zone (26) when occupied by a first user;
determining, from the first volume indicator, a first operational parameter for thermoregulation fluid to be provided for treating the first user;

**characterized in that** the first operational parameter is a volumetric parameter.

2. A method according to claim 1, wherein the first operational parameter is determined directly from a function of the first volume indicator and/or in dependence on a treatment type.

3. A method according to any preceding claim, wherein the first operational parameter is a first volume of thermoregulation fluid to be provided for treating the first user.

4. A method according to any preceding claim, wherein the pack (14, 200) includes a pressurising element (22, 226) for receiving pressure fluid and for applying pressure to the treatment zone (26), and obtaining the first volume indicator includes:

obtaining a target pressure fluid pressure and a reference pressure fluid volume, wherein the reference pressure fluid volume is a volume of pressure fluid that would cause the pressurising element (22, 226) to be at the target pressure fluid pressure with the treatment zone (26) unoccupied; and
performing a first volume indicator determination procedure including:

operating the pressure fluid supply system to supply pressure fluid to the pressurising element (22, 226) with the first user occupying the treatment zone (26), whereby to increase the pressure of pressure fluid in the pressurising element (22, 226);
detecting the pressure of pressure fluid in the pressurising element (22, 226) reaching the target pressure fluid pressure;
in response to detection of the pressure of pressure fluid in the pressurising element (22, 226) reaching the target pressure fluid pressure, determining a first volume of pressure fluid, the first volume of pressure fluid being a volume of pressure fluid that has caused the pressure of pressure fluid in the pressurising element (22, 226) to reach the target pressure fluid pressure;
determining a volume difference by subtracting the first volume of pressure fluid from the reference pressure fluid volume; and
determining the first volume indicator from the volume difference.

5. A method according to claim 4, wherein obtaining a target pressure fluid pressure and a reference pressure fluid volume includes:

obtaining data representing a type of pack to be used;

accessing a data repository containing a plurality of pack data sets, each pack data set including a target pressure fluid pressure and a reference pressure fluid volume for a different type of pack;

obtaining from the data repository the pack data set corresponding to the type of pack to be used.

6. A program for performing the method of any preceding claim when executed on an operation unit of a thermoregulation system.

7. An operation unit, the operation unit being for a personal thermoregulation system for being coupled to, and providing a treatment to a user via, a personal thermoregulation pack (14, 200) which includes a thermoregulation zone for at least partially surrounding a treatment zone (26) and for applying a thermal treatment to a user at the treatment zone (26), the operation unit being operable to:

measure a first volume indicator, the first volume indicator being indicative of a volume of a treatment zone (26) of a first pack when occupied by a first user;

determine, from the first volume indicator, a first operational parameter for thermoregulation fluid to be provided for treating the first user with the first pack;

**characterized in that** the first operational parameter is a volumetric parameter.

8. A personal thermoregulation system for being coupled to, and providing a treatment to a user via, a personal thermoregulation pack (14, 200) which includes a thermoregulation zone for at least partially surrounding a treatment zone (26) and for applying a thermal treatment to a user at the treatment zone (26), the thermoregulation system including an operation unit according to claim 7.

9. A thermoregulation system according to claim 8, wherein the first operational parameter is a first volume of thermoregulation fluid to be provided for treating the first user.

10. A thermoregulation system according to claim 9, wherein the thermoregulation system includes a thermoregulation fluid supply system for supplying thermoregulation fluid to a coupled pack (14, 200), and the operation unit is operable to operate the thermoregulation fluid supply system to supply thermoregulation fluid to a coupled pack to provide the first volume of thermoregulation fluid.

11. A thermoregulation system according to any of claims 8 to 10, including a pressure fluid supply system for supplying pressure fluid to a pressurising element (22, 226) of a coupled pack, a pressure obtaining element for obtaining a determination of a pressure of pressure fluid in a pressurising element (22, 226) of a coupled pack, and a pressure fluid volume determination obtaining element for obtaining a determination of a pressure fluid volume, wherein the operation unit is operable to:

obtain a target pressure fluid pressure and a reference pressure fluid volume, wherein the reference pressure fluid volume is a volume of pressure fluid that would cause the pressurising element (22, 226) of the first pack to be at the target pressure fluid pressure with the treatment zone (26) unoccupied; and

perform a first volume indicator determination procedure, including:

operating the pressure fluid supply system to supply pressure fluid to the pressurising element (22, 226) of a coupled pack whereby to increase the pressure of pressure fluid in the pressurising element (22, 226) of the coupled pack;

obtaining via the pressure obtaining element a determination of a pressure of pressure fluid in the pressurising element (22, 226) of the coupled pack;

detecting a pressure of pressure fluid in the pressurising element (22, 226) of the coupled pack reaching the target pressure fluid pressure;

in response to detecting the pressure of pressure fluid in the pressurising element (22, 226) of the coupled pack reaching the target pressure fluid pressure, obtaining, via the pressure fluid volume determination obtaining element, a determination of a first volume of pressure fluid, the first volume of pressure fluid being a volume of pressure fluid that has caused the pressure of pressure fluid in the pressurising element (22, 226) of the coupled pack to reach the target pressure fluid pressure;

determining a volume difference by subtracting the first volume of pressure fluid from the reference pressure fluid volume; and

determining the first volume indicator from the volume difference.

12. A thermoregulation system according to claim 11, including a pack data obtaining element for obtaining data representing a type of pack to be used, wherein the operation unit is operable to:

access a data repository containing a plurality of pack data sets, each pack data set including a target pressure fluid pressure and a reference pressure fluid volume for a different type of pack; obtain from the data repository the pack data set corresponding to the type of pack to be used.

13. A thermoregulation system according to any of claims 8 to 12, including:

an output port for coupling to an input of a personal thermoregulation pack;
a thermoregulation fluid supply system for supplying thermoregulation fluid to the output port;
an output pump for pumping thermoregulation fluid out through the output port; and
a pressure control system for selectively operating a pressurising element of a personal thermoregulation pack to apply a pressure independently of operation of the output pump.

14. A thermoregulation system according to claim 13, including:

an input port for coupling to an output of a personal thermoregulation pack; and
an input pump for pumping thermoregulation fluid in through the input port;
wherein the pressure control system is for operating a pressurising element of a personal thermoregulation pack to apply a pressure independently of operation of the input pump.

15. A personal thermoregulation assembly, including:

a personal thermoregulation system according to any of claims 8 to 14; and
a personal thermoregulation pack (14, 200) for being placed against a user and coupled to the personal thermoregulation system for providing a treatment to a user.

**Patentansprüche**

1. Verfahren zum Bestimmen eines Betriebsparameters für ein Thermoregulationsfluid, das für eine persönliche Thermoregulationspackung (14, 200) bereitzustellen ist, wobei die Packung eine Thermoregulationszone zum mindestens teilweise Umgeben einer Behandlungszone (26) und zum Anwenden einer Wärmebehandlung auf einen Nutzer an der Behandlungszone (26) aufweist, wobei das Verfahren aufweist:

Messen eines ersten Volumenindikators, wobei der erste Volumenindikator ein Volumen der Behandlungszone (26) angibt, wenn sie von einem ersten Nutzer besetzt ist;
Bestimmen, aus dem ersten Volumenindikator, eines ersten Betriebsparameters für Thermoregulationsfluid das zur Behandlung des ersten Nutzers bereitgestellt wird;
**dadurch gekennzeichnet, dass**
der erste Betriebsparameter ein volumetrischer Parameter ist.

2. Verfahren gemäß Anspruch 1, wobei der erste Betriebsparameter direkt aus einer Funktion des ersten Volumenindikators und/oder in Abhängigkeit von einer Art der Behandlung bestimmt wird.

3. Verfahren gemäß einem der vorhergehenden Ansprüche, wobei der erste Betriebsparameter ein erstes Volumen von Thermoregulationsfluid ist, das zur Behandlung des ersten Nutzers bereitgestellt wird.

4. Verfahren gemäß einem der vorhergehenden Ansprüche, wobei die Packung (14, 200) ein Druckausübungselement (22, 226) zum Aufnehmen von Druckfluid und zum Ausüben von Druck auf die Behandlungszone (26) aufweist, und das Beschaffen des ersten Volumenindikators aufweist:

Beschaffen eines Zieldruck-Fluiddrucks und eines Referenzdruck-Fluidvolumens, wobei das Referenzdruck-Fluidvolumen ein Volumen von Druckfluid ist, das verursachen würde, dass das Druckausübungselement (22, 226) mit dem Zieldruck-Fluiddruck beaufschlagt ist, während die Behandlungszone (26) unbesetzt ist; und
Durchführen eines ersten Volumenindikator-Bestimmungsvorgangs, der aufweist:

Betreiben des Druckfluid-Zufuhrsystems zum Zuführen von Druckfluid an das Druckausübungselement (22, 226), wobei der erste Nutzer die Behandlungszone (26) besetzt, wodurch der Druck von Druckfluid in dem Druckausübungselement (22, 226) erhöht wird;
Erfassen, dass der Druck von Druckfluid in dem Druckausübungselement (22, 226) den Zieldruck-Fluiddruck erreicht;
in Reaktion darauf, dass erfasst wird, dass der Druck von Druckfluid in dem Druckausübungselement (22, 226) den Zieldruck-Fluiddruck erreicht, Bestimmen eines ersten Volumens von Druckfluid, wobei das erste Volumen von Druckfluid ein Volumen von Druckfluid ist, das verursacht hat, dass der Druck von Druckfluid in dem Druckausübungselement (22, 226) den Zieldruck-Fluiddruck erreicht;
Bestimmen einer Volumendifferenz durch Abziehen des ersten Volumens von Druckfluid von dem Referenzdruck-Fluidvolumen; und

Bestimmen des ersten Volumenindikators aus der Volumendifferenz.

5. Verfahren gemäß Anspruch 4, wobei das Beschaffen eines Zieldruck-Fluiddrucks und eines Referenzdruck-Fluidvolumens aufweist:

Beschaffen von Daten, die einen zu verwendenden Packungstyp repräsentieren;
Zugreifen auf einen Datenspeicher, der mehrere Packungs-Datensätze enthält, wobei jeder Packungs-Datensatz einen Zieldruck-Fluiddruck und ein Referenzdruck-Fluidvolumen für einen unterschiedlichen Packungstyp beinhaltet;
Beschaffen des Packungs-Datensatzes, der dem zu verwendenden Packungstyp entspricht, von dem Datenspeicher.

6. Programm zum Durchführen des Verfahrens gemäß einem der vorhergehenden Ansprüche, wenn es auf einer Betriebseinheit eines Thermoregulationssystems ausgeführt wird.

7. Betriebseinheit, wobei die Betriebseinheit für ein persönliches Thermoregulationssystem ist, das dazu dient, über eine persönliche Thermoregulationspackung (14, 200) mit einem Nutzer gekoppelt zu werden und diesem eine Behandlung zukommen zu lassen, wobei die Thermoregulationspackung (14, 200) eine Thermoregulationszone zum mindestens teilweise Umgeben einer Behandlungszone (26) und zum Anwenden einer Wärmebehandlung auf einen Nutzer an der Behandlungszone (26) aufweist, wobei die Betriebseinheit betreibbar ist zum:

Messen eines ersten Volumenindikators, wobei der erste Volumenindikator ein Volumen der Behandlungszone (26) einer ersten Packung angibt, wenn sie von einem ersten Nutzer besetzt ist;
Bestimmen, aus dem ersten Volumenindikator, eines ersten Betriebsparameters für Thermoregulationsfluid das zur Behandlung des ersten Nutzers mit der ersten Packung bereitgestellt wird;
**dadurch gekennzeichnet, dass**
der erste Betriebsparameter ein volumetrischer Parameter ist.

8. Persönliches Thermoregulationssystem, das dazu dient, über eine persönliche Thermoregulationspackung (14, 200) mit einem Nutzer gekoppelt zu werden und diesem eine Behandlung zukommen zu lassen, wobei die Thermoregulationspackung (14, 200) eine Thermoregulationszone zum mindestens teilweise Umgeben einer Behandlungszone (26) und zum Anwenden einer Wärmebehandlung auf einen Nutzer an der Behandlungszone (26) aufweist, wobei das Thermoregulationssystem eine Betriebseinheit gemäß Anspruch 7 aufweist.

9. Thermoregulationssystem gemäß Anspruch 8, wobei der erste Betriebsparameter ein erstes Volumen von Thermoregulationsfluid ist, das zur Behandlung des ersten Nutzers bereitgestellt wird.

10. Thermoregulationssystem gemäß Anspruch 9, wobei das Thermoregulationssystem ein Thermoregulationsfluid-Zufuhrsystem zum Zuführen von Thermoregulationsfluid an eine angeschlossene Packung (14, 200) aufweist und die Betriebseinheit dazu betreibbar ist, das Thermoregulationsfluid-Zufuhrsystem zu betreiben, sodass es Thermoregulationsfluid an eine angeschlossene Packung zuführt, um das erste Volumen von Thermoregulationsfluid bereitzustellen.

11. Thermoregulationssystem gemäß einem der Ansprüche 8 bis 10, aufweisend ein Druckfluid-Zufuhrsystem zum Zuführen von Druckfluid an ein Druckausübungselement (22, 226) einer angeschlossenen Packung, ein Druckbeschaffungselement zum Beschaffen einer Bestimmung eines Drucks von Druckfluid in einem Druckausübungselement (22, 226) einer angeschlossenen Packung, und ein Druckfluid-Volumenbestimmungs-Beschaffungselement zum Beschaffen einer Bestimmung eines Druckfluidvolumens, wobei die Betriebseinheit betreibbar ist zum:

Beschaffen eines Zieldruck-Fluiddrucks und eines Referenzdruck-Fluidvolumens, wobei das Referenzdruck-Fluidvolumen ein Volumen von Druckfluid ist, das verursachen würde, dass das Druckausübungselement (22, 226) mit dem Zieldruck-Fluiddruck beaufschlagt ist, während die Behandlungszone (26) unbesetzt ist; und
Durchführen eines ersten Volumenindikator-Bestimmungsvorgangs, der aufweist:

Betreiben des Druckfluid-Zufuhrsystems zum Zuführen von Druckfluid an das Druckausübungselement (22, 226) einer angeschlossenen Packung, wodurch der Druck von Druckfluid in dem Druckausübungselement (22, 226) erhöht wird;
Beschaffen, über das Druckbeschaffungselement, einer Bestimmung eines Drucks von Druckfluid in dem Druckausübungselement (22, 226) der angeschlossenen Packung;
Erfassen, dass ein Druck von Druckfluid in dem Druckausübungselement (22, 226) den Zieldruck-Fluiddruck erreicht;

in Reaktion darauf, dass erfasst wird, dass der Druck von Druckfluid in dem Druckausübungselement (22, 226) den Zieldruck-Fluiddruck erreicht, Beschaffen, über das Druckfluid-Volumenbestimmungs-Beschaffungselement, einer Bestimmung eines ersten Volumens von Druckfluid, wobei das erste Volumen von Druckfluid ein Volumen von Druckfluid ist, das verursacht hat, dass der Druck von Druckfluid in dem Druckausübungselement (22, 226) der angeschlossenen Packung den Zieldruck-Fluiddruck erreicht;

Bestimmen einer Volumendifferenz durch Abziehen des ersten Volumens von Druckfluid von dem Referenzdruck-Fluidvolumen; und

Bestimmen des ersten Volumenindikators aus der Volumendifferenz.

12. Thermoregulationssystem gemäß Anspruch 11, aufweisend ein Packungsdaten-Beschaffungselement zum Beschaffen von Daten, die den zu verwendenden Packungstyp repräsentieren, wenn die Betriebseinheit betreibbar ist zum:

Zugreifen auf einen Datenspeicher, der mehrere Packungs-Datensätze enthält, wobei jeder Packungs-Datensatz einen Zieldruck-Fluiddruck und ein Referenzdruck-Fluidvolumen für einen unterschiedlichen Packungstyp beinhaltet;

Beschaffen des Packungs-Datensatzes, der dem zu verwendenden Packungstyp entspricht, von dem Datenspeicher.

13. Thermoregulationssystem gemäß einem der Ansprüche 8 bis 12, aufweisend:

einen Ausgangsanschluss zum Verbinden mit einem Eingang einer persönlichen Thermoregulationspackung;

ein Thermoregulationsfluid-Zufuhrsystem zum Zuführen von Thermoregulationsfluid an den Ausgangsanschluss;

eine Ausgangspumpe zum Pumpen von Thermoregulationsfluid aus dem Ausgangsanschluss heraus; und

ein Druckregelungssystem zum selektiven Betreiben eines Druckausübungselements einer persönlichen Thermoregulationspackung zum Anlegen eines Drucks unabhängig von einem Betrieb der Ausgangspumpe.

14. Thermoregulationssystem gemäß Anspruch 13, aufweisend:

einen Eingangsanschluss zum Verbinden mit einem Ausgang einer persönlichen Thermoregulationspackung; und

eine Eingangspumpe zum Pumpen von Thermoregulationsfluid in den Eingangsanschluss hinein;

wobei das Druckregelungssystem zum Betreiben eines Druckausübungssystems einer persönlichen Thermoregulationspackung dient, um Druck unabhängig von einem Betrieb der Ausgangspumpe anzulegen.

15. Persönliche Thermoregulationsanordnung, aufweisend:

ein persönliches Thermoregulationssystem gemäß einem der Ansprüche 8 bis 14; und

eine persönliche Thermoregulationspackung (14, 200), die dazu gedacht ist, an einen Nutzer angelegt zu werden und an das persönliche Thermoregulationssystem angeschlossen zu werden, um einem Nutzer eine Behandlung zukommen zu lassen.

## Revendications

1. Procédé de détermination d'un paramètre fonctionnel pour un fluide de thermorégulation à fournir pour une enveloppe de traitement de thermorégulation personnelle (14, 200), l'enveloppe comprenant une zone de thermorégulation pour entourer au moins partiellement une zone de traitement (26) et pour appliquer un traitement thermique à un utilisateur au niveau de la zone de traitement (26), le procédé comprenant :

mesurer un premier indicateur de volume, le premier indicateur de volume étant indicateur d'un volume de la zone de traitement (26) lorsqu'occupée par un premier utilisateur ;

déterminer, à partir du premier indicateur de volume, un premier paramètre fonctionnel pour le fluide de thermorégulation à fournir pour traiter le premier utilisateur ; **caractérisé par le fait que** le premier paramètre fonctionnel est un paramètre volumétrique.

2. Procédé selon la revendication 1, dans lequel le premier paramètre fonctionnel est déterminé directement à partir d'une fonction du premier indicateur de volume et/ou en dépendance d'un type de traitement.

3. procédé selon l'une quelconque des revendications précédentes, dans lequel le premier paramètre fonctionnel est un premier volume de fluide de thermorégulation à fournir pour traiter le premier utilisateur.

4. Procédé selon l'une quelconque des revendications précédentes, dans lequel l'enveloppe (14, 200) comprend un élément de mise sous pression (22, 226) pour recevoir du fluide de pression et pour appliquer une pression à la zone de traitement (26), et obtenir le premier indicateur de volume comprend :

obtenir une pression cible de fluide de pression et un volume de référence de fluide de pression, le volume de référence de fluide de pression étant un volume de fluide de pression qui amènerait l'élément de mise sous pression (22, 226) à être à la pression cible de fluide de pression avec la zone de traitement (26) non occupée ; et réaliser une procédure de détermination de premier indicateur de volume comprenant :

faire fonctionner le système de distribution de fluide de pression pour distribuer du fluide de pression à l'élément de mise sous pression (22, 226) avec le premier utilisateur occupant la zone de traitement (26), de façon à ainsi augmenter la pression du fluide de pression dans l'élément de mise sous pression (22, 226) ;
détecter la pression du fluide de pression dans l'élément de mise sous pression (22, 226) atteignant la pression cible de fluide de pression ;
en réponse à la détection de la pression du fluide de pression dans l'élément de mise sous pression (22, 226) atteignant la pression cible de fluide de pression, déterminer un premier volume de fluide de pression, le premier volume de fluide de pression étant un volume de fluide de pression qui a amené la pression du fluide de pression dans l'élément de mise sous pression (22, 226) à atteindre la pression cible de fluide de pression ;
déterminer une différence de volume par soustraction du premier volume de fluide de pression à partir du volume de référence de fluide de pression ; et
déterminer le premier indicateur de volume à partir de la différence de volume.

5. Procédé selon la revendication 4, dans lequel obtenir une pression cible de fluide de pression et un volume de référence de fluide de pression comprend :

obtenir des données représentant un type d'enveloppe à utiliser ;
accéder à un dépôt de données contenant une pluralité d'ensembles de données d'enveloppe, chaque ensemble de données d'enveloppe comprenant une pression cible de fluide de pression et un volume de référence de fluide de pression pour un type différent d'enveloppe ;

obtenir à partir du dépôt de données l'ensemble de données d'enveloppe correspondant au type d'enveloppe à utiliser.

6. Programme pour réaliser le procédé selon l'une quelconque des revendications précédentes lorsqu'exécuté sur une unité fonctionnelle d'un système de thermorégulation.

7. Unité fonctionnelle, l'unité fonctionnelle étant pour un système de thermorégulation personnel destiné à être couplé à, et à fournir un traitement à un utilisateur par l'intermédiaire de, une enveloppe de thermorégulation personnelle (14, 200) qui comprend une zone de thermorégulation pour entourer au moins partiellement une zone de traitement (26) et pour appliquer un traitement thermique à un utilisateur au niveau de la zone de traitement (26), l'unité fonctionnelle étant actionnable pour :

mesurer un premier indicateur de volume, le premier indicateur de volume étant indicateur d'un volume d'une zone de traitement (26) d'une première enveloppe lorsqu'occupée par un premier utilisateur ;
déterminer, à partir du premier indicateur de volume, un premier paramètre fonctionnel pour un fluide de thermorégulation à fournir pour traiter le premier utilisateur avec la première enveloppe ; **caractérisée par le fait que** le premier paramètre fonctionnel est un paramètre volumétrique.

8. Système de thermorégulation personnel destiné à être couplé à, et à fournir un traitement à un utilisateur par l'intermédiaire de, une enveloppe de thermorégulation personnelle (14, 200) qui comprend une zone de thermorégulation pour entourer au moins partiellement une zone de traitement (26) et pour appliquer un traitement thermique à un utilisateur au niveau de la zone de traitement (26), le système de thermorégulation comprenant une unité fonctionnelle selon la revendication 7.

9. Système de thermorégulation selon la revendication 8, dans lequel le premier paramètre fonctionnel est un premier volume de fluide de thermorégulation à fournir pour traiter le premier utilisateur.

10. Système de thermorégulation selon la revendication 9, dans lequel le système de thermorégulation comprend un système de distribution de fluide thermorégulation pour distribuer du fluide de thermorégulation à une enveloppe couplée (14, 200), et l'unité fonctionnelle étant actionnable pour faire fonctionner le système de distribution de fluide thermorégulation pour distribuer du fluide thermorégulation à une enveloppe couplée pour fournir le premier volume de fluide thermorégulation.

**11.** Système de thermorégulation selon l'une quelconque des revendications 8 à 10, comprenant un système de distribution de fluide de pression pour distribuer du fluide de pression à un élément de mise sous pression (22, 226) d'une enveloppe couplée, un élément d'obtention de pression pour obtenir une détermination d'une pression d'un fluide de pression dans un élément de mise sous pression (22, 226) d'une enveloppe couplée, et un élément d'obtention de détermination de volume de fluide de pression pour obtenir une détermination d'un volume de fluide de pression, l'unité fonctionnelle étant actionnable pour :

obtenir une pression cible de fluide de pression et un volume de référence de fluide de pression, le volume de référence de fluide de pression étant un volume de fluide de pression qui amènerait l'élément de mise sous pression (22, 226) de la première enveloppe à être à la pression cible de fluide de pression avec la zone de traitement (26) non occupée ; et

réaliser une procédure de détermination de premier indicateur de volume, comprenant :

faire fonctionner le système de distribution de fluide de pression pour distribuer du fluide de pression à l'élément de mise sous pression (22, 226) d'une enveloppe couplée, de façon à ainsi augmenter la pression du fluide de pression dans l'élément de mise sous pression (22, 226) de l'enveloppe couplée ;

obtenir par l'intermédiaire de l'élément d'obtention de pression une détermination d'une pression du fluide de pression dans l'élément de mise sous pression (22, 226) de l'enveloppe couplée ;

détecter une pression du fluide de pression dans l'élément de mise sous pression (22, 226) de l'enveloppe couplée atteignant la pression cible de fluide de pression ;

en réponse à la détection de la pression du fluide de pression dans l'élément de mise sous pression (22, 226) de l'enveloppe couplée atteignant la pression cible de fluide de pression, obtenir, par l'intermédiaire de l'élément d'obtention de détermination de volume de fluide de pression, une détermination d'un premier volume de fluide de pression, le premier volume de fluide de pression étant un volume de fluide de pression qui a amené la pression du fluide de pression dans l'élément de mise sous pression (22, 226) de l'enveloppe couplée à atteindre la pression cible de fluide de pression ;

déterminer une différence de volume par soustraction du premier volume de fluide de pression à partir du volume de référence de fluide de pression ; et

déterminer le premier indicateur de volume à partir de la différence de volume.

**12.** Système de thermorégulation selon la revendication 11, comprenant un élément d'obtention de données d'enveloppe pour obtenir des données représentant un type d'enveloppe à utiliser, l'unité fonctionnelle étant actionnable pour :

accéder à un dépôt de données contenant une pluralité d'ensembles de données d'enveloppe, chaque ensemble de données d'enveloppe comprenant une pression cible de fluide de pression et un volume de référence de fluide de pression pour un type différent d'enveloppe ;

obtenir à partir du dépôt de données l'ensemble de données d'enveloppe correspondant au type d'enveloppe à utiliser.

**13.** Système de thermorégulation selon l'une quelconque des revendications 8 à 12, comprenant :

un orifice de sortie pour couplage à une entrée d'une enveloppe de thermorégulation personnelle ;

un système de distribution de fluide de thermorégulation pour distribuer du fluide thermorégulation à l'orifice de sortie ;

une pompe de sortie pour pomper du fluide thermorégulation vers l'extérieur à travers l'orifice de sortie ; et

un système de commande de pression pour actionner de manière sélective un élément de mise sous pression d'une enveloppe de thermorégulation personnelle pour appliquer une pression indépendamment du fonctionnement de la pompe de sortie.

**14.** Système de thermorégulation selon la revendication 13, comprenant :

un orifice d'entrée couplage à une sortie d'une enveloppe de thermorégulation personnelle ; et

une pompe d'entrée pour pomper du fluide de thermorégulation vers l'intérieur à travers l'orifice d'entrée ;

le système de commande de pression servant à actionner un élément de mise sous pression d'une enveloppe de thermorégulation personnelle pour appliquer une pression indépendamment du fonctionnement de la pompe d'entrée.

**15.** Ensemble de thermorégulation personnel, comprenant :

un système de thermorégulation personnel selon l'une quelconque des revendications 8 à 14 ; et
une enveloppe de thermorégulation personnelle (14, 200) destinée à être placée contre un utilisateur et à être couplée au système de thermorégulation personnel pour fournir un traitement à un utilisateur.

# Figure 1

Figure 2

Figure 3

# Figure 4

## Cuff circulation

# Figure 5

## Internal circulation

# Figure 6

Internal circulation and
Cuff circulation

# Figure 7

## Circulation via hot /cold tanks

Figure 8

# Figure 9

# Figure 10a

# Figure 10b

# Figure 10c

210

210

222

226

AIR
FLOW

230

240

EP 3 021 802 B1

# Figure 10d

40

# Figure 11

4 - Pump System

# Figure 12

## Pack Circulation

# Figure 13

## Internal Circulation

# Figure 14

## Internal Circulation and Pack Circulation

# Figure 15

HOT TANK

66

V3

V1

V4

V8

COLD TANK

V2

68

312

89

90

67

314

V7

V6

14

## Circulation via Hot / Cold Tanks

Figure 16

# Figure 17

## Hot Tank Internal Circulation

# Figure 18

# Figure 19

| Air Pressure = 30mmHg | Air Pressure = 30mmHg | |
| Air Volume = 1000ml | Air Volume = 300ml | Limb Volume = 700ml |
| Max Air Volume | Current Air Volume | Limb Volume |
| a | b | c |

# Figure 20

LIMB 'CONTACT' VOLUME

LIMB

PACK AIR VOLUME

# Figure 21

|   |   | USER 1 | USER 2 |   |
|---|---|---|---|---|
| A | Pack Specific - Limb Volume Datum | 500ml | | |
| B | Pack Coolant Volume Datum | 250ml | | |
| C | Pack Limb to Coolant Multiple Factor | 0.5 | | B / A = C |
| D | Pack Max Air Volume | 1000ml | | |
| E | Pack Tune Air Volume | 600ml | 400ml | |
| F | Limb Volume | 400ml | 600ml | |
| G | Pack Coolant Volume Post-Adjustment | 200ml | 300ml | F x C = G |
| H | Deep Tissue Trauma Multiple Factor | 1.5 | | |
| I | Post Activity Cool Down Multiple Factor | 0.5 | | |
| J | Deep Tissue Trauma Coolant Volume | 300ml | 450ml | G x H = J |
| K | Post Activity Cool Down Coolant Volume | 100ml | 150ml | G x I = K |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013190337 A
- WO 2013190336 A
- US 20050103353 A
- WO 2014188213 A